# EUROPEAN PATENT SPECIFICATION

(11) **EP 4 067 905 B1**
(45) Date of publication and mention of the grant of the patent: **09.09.2026**
(21) Application number: 20892121.3
(22) Date of filing: 27.11.2020
(51) Int. Cl.: G01N 33/92, G01N 33/68

(54) **METHOD FOR ASSISTING DIAGNOSIS OF PARKINSON'S DISEASE, BIOMARKER, REAGENT KIT, AND DEVICE**
VERFAHREN ZUR UNTERSTÜTZUNG DER DIAGNOSE VON MORBUS PARKINSON, BIOMARKER, REAGENZKIT UND VORRICHTUNG
PROCÉDÉ D'AIDE AU DIAGNOSTIC DE LA MALADIE DE PARKINSON, BIOMARQUEUR, KIT DE RÉACTIFS ET DISPOSITIF

(30) Priority: 29.11.2019 JP 2019217481
(43) Date of publication of application: 05.10.2022
(73) Proprietor: FUJIFILM Wako Pure Chemical Corporation, Osaka 540-8605 (JP)
(72) Inventor: NISHIBU, Takahiro, Amagasaki-shi, Hyogo 661-0963 (JP); IMAWAKA, Naoko, Amagasaki-shi, Hyogo 661-0963 (JP); HIRAYASU, Kazunari, Amagasaki-shi, Hyogo 661-0963 (JP); UKEKAWA, Ryo, Amagasaki-shi, Hyogo 661-0963 (JP); SASAMOTO, Kodai, Amagasaki-shi, Hyogo 661-0963 (JP); ONODERA, Satoshi, Shibukawa-shi, Gunma 377-0007 (JP)
(74) Representative: Klunker IP Patentanwälte PartG mbB
(86) International application number: PCT/JP2020/044400
(87) International publication number: WO 2021/107155

(56) References cited:
- EP-A1- 3 228 709
- EP-A1- 4 067 906
- WO-A1-2016/088689
- WO-A1-2017/032871
- WO-A1-2018/145211
- WO-A1-2018/218090
- CA-A1- 2 941 460
- JP-A- 2016 161 480
- JP-A- 2017 525 976
- US-A1- 2013 337 440
- US-A1- 2018 067 134
- US-A1- 2018 080 945
- NAKAI WATARU ET AL: "A novel affinity-based method for the isolation of highly purified extracellular vesicles", vol. 6, no. 33935, 23 September 2016 (2016-09-23), pages 1 - 11, XP055842512, Retrieved from the Internet <URL:https://www.nature.com/articles/srep33935.pdf> DOI: 10.1038/srep33935
- LAMONTAGNE-PROULX JÉRÔME ET AL: "Portrait of blood-derived extracellular vesicles in patients with Parkinson's disease", NEUROBIOLOGY OF DISEASE, vol. 124, 1 April 2019 (2019-04-01), AMSTERDAM, NL, pages 163 - 175, XP093010548, ISSN: 0969-9961, DOI: 10.1016/j.nbd.2018.11.002
- GREY MARIE ET AL: "Acceleration of [alpha]-Synuclein Aggregation by Exosomes", JOURNAL OF BIOLOGICAL CHEMISTRY, vol. 290, no. 5, 1 January 2015 (2015-01-01), US, pages 2969 - 2982, XP093010541, ISSN: 0021-9258, DOI: 10.1074/jbc.M114.585703
- OHMICHI, TAKUMA ET AL.: "Quantification of brain- derived extracellular vesicles in plasma as a biomarker to diagnose Parkinson's diseases", PARKINSONISM AND RELATED DISORDERS, vol. 61, April 2019 (2019-04-01), pages 82 - 87, XP085669225, DOI: 10.1016/j.parkreldis.2018.11.021

## Description

### Technical Field

The present invention relates to a method for assisting the diagnosis of Parkinson's disease, use of a biomarker, and use of a reagent kit.

### Background Art

Parkinson's disease (PD) is a neurodegenerative disease in which nerve cells existing in the substantia nigra in the brain are degenerated and the striatal dopamine is deficient, which makes it impossible for the body to move smoothly. The prevalence of Parkinson's disease is as high as 100 to 150 per 100,000 and increases with aging, so the number of patients with Parkinson's disease is increasing rapidly with the aging of the population.

Currently, there is no cure for Parkinson's disease, but it is known that motor symptoms can be improved by administering a dopamine precursor therapeutic agent such as levodopa (L-dopa) or a dopamine agonist to supplement the reduced dopaminergic effects in the brain. Therefore, there is a demand for a biomarker that can objectively determine the pathological condition of Parkinson's disease.

A method of concentrating nerve-derived extracellular vesicles in blood using an anti-NCAM antibody or an anti-L1CAM antibody, and determining Parkinson's disease using α-synuclein or the like contained in the extracellular vesicles as an indicator has been reported as a method for the diagnosis of Parkinson's disease (Patent Literature 1, Patent Literature 2, Non-Patent Literature 1, and Non-Patent Literature 2).

Patent Literature 3 discloses a method for assisting the diagnosis of Alzheimer's disease or mild cognitive impairment, a biomarker, a reagent kit and a device.

Patent Literature 4 and Non-Patent Literature 3 disclose an affinity-based method for the isolation of highly purified extracellular vesicles, especially, methods for obtaining, removing and detecting extracellular membrane vesicles using a Tim protein-bonding carrier.

Patent Literature 5, Patent Literature 6 and Non-Patent Literature 4 disclose a method for clinically assessing Parkinson's disease in a human subject using erythrocyte-derived extracellular vesicles (EEV) as a biomarker. The values in items of DC235a (glycophorin A) positive and PS (phosphatidylserine) negative, DC235a positive and total and EV (extracellular vesicles) DC235a positive/erythrocyte have significance difference between Parkinson's disease patients and controls (ex. Table 2A of Patent Literature 5).

Non-Patent Literature 5 discloses that exosomes accelerate the aggregation of alpha-synuclein in Parkinson's disease.

### Citation List

### Patent Literature

Patent Literature 1: JP2016-550673
Patent Literature 2: JP2017-520760
Patent Literature 3: EP 4 067 906
Patent Literature 4: EP 3 228 709
Patent Literature 5: CA 2 941 460
Patent Literature 6: US 2018/067134

### Non-Patent Literature

Non-Patent Literature 1: C.N. Winston et al./Alzheimer's & Dementia: Diagnosis, Assessment & Disease Monitoring (2016) 3:63-72
Non-Patent Literature 2: Mustapic M et al. Front. Neurosci. (2017) 11:278
Non-Patent Literature 3: W. Nakai et al., SCIENTIFIC REPORTS, vol. 6, no. 33935, pages 1 - 11 (2016.09.23)
Non-Patent Literature 4: J. Lemontagne-Proulx et al., NEUROBIOLOGY OF DISEASE, AMSTERDAM, NL, vol. 124, pages 163 - 175 (2019.04.01)
Non-Patent Literature 5: M. Grey et al., JOURNAL OF BIOLOGICAL CHEMISTRY, US, vol. 290, no. 5, pages 2969 - 2982 (2015.01.30)

### Summary of Invention

### Technical Problem

However, the method for the diagnosis of Parkinson's disease using nerve-derived extracellular vesicles in blood as an indicator requires an operation of affinity concentration of the nerve-derived extracellular vesicles from a test specimen, and the affinity concentration operation is complicated. In addition, an error is likely to occur between the assays since the test specimen cannot be measured directly, and it is difficult to apply such a diagnosis method to the measurement of multiple test specimens.

The present invention has been conceived in view of the above circumstances, and an object of the present invention is to provide a method for easily assisting the diagnosis of Parkinson's disease, use of a biomarker, and use of a reagent kit.

### Solution to Problem

The present inventors examined whether a specific extracellular vesicle could be a biomarker to assist in the diagnosis of Parkinson's disease.

As a result, the present inventors have newly found that a specific extracellular vesicle having phosphatidylserine and tetraspanin, among extracellular vesicles, serves as a biomarker for assisting the diagnosis of Parkinson's disease. The present invention has been completed based on these findings.

The present invention relates to a method for assisting the diagnosis of Parkinson's disease, use of a biomarker, and a reagent kit as defined in the appended claims.

### Advantageous Effects of Invention

According to the present invention, it is possible to easily assist in the diagnosis of Parkinson's disease.

### (Brief Description of Drawings)

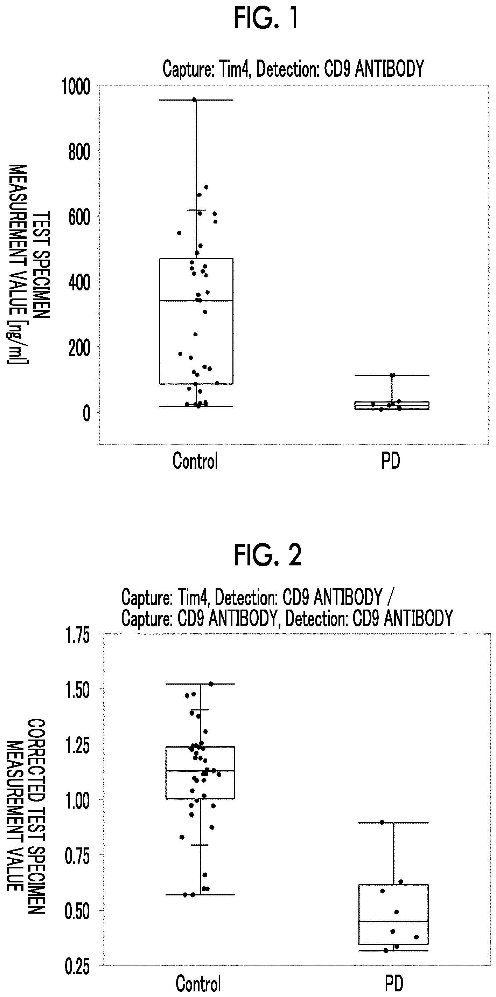

Fig. 1 is a box plot graph of evaluation results of a plasma test specimen derived from a patient with Parkinson's disease without cognitive symptoms and a plasma test specimen derived from a healthy subject using an amount of exosomes having phosphatidylserine and CD9 as an indicator.
Fig. 2 is a box plot graph of evaluation results of a plasma test specimen derived from a patient with Parkinson's disease without cognitive symptoms and a plasma test specimen derived from a healthy subject using a ratio of an amount of exosomes having phosphatidylserine and CD9 to an amount of extracellular vesicles having CD9 as an indicator.

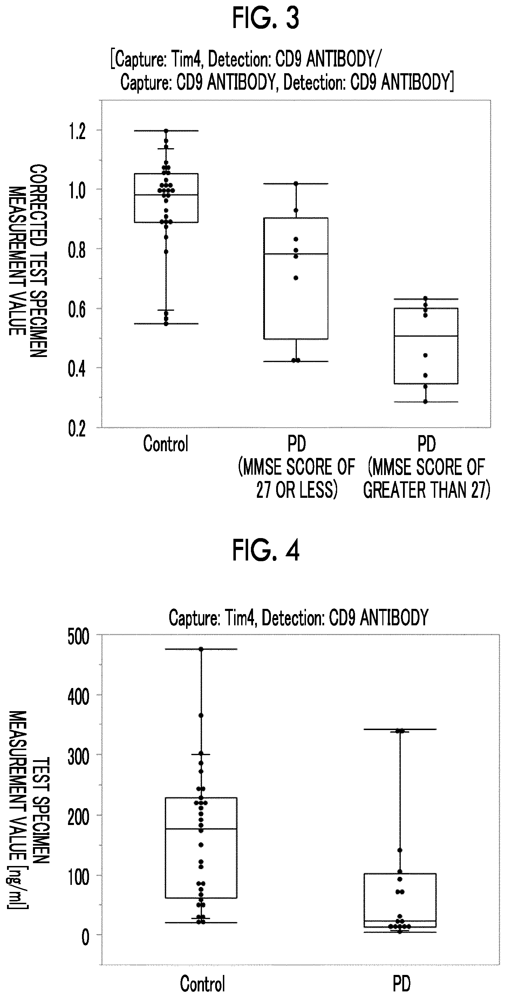

Fig. 3 is a box plot graph of evaluation results of a plasma test specimen derived from a patient with Parkinson's disease without cognitive symptoms, a plasma test specimen derived from a patient with Parkinson's disease with cognitive symptoms, and a plasma test specimen derived from a healthy subject using a ratio of an amount of exosomes having phosphatidylserine and CD9 to an amount of extracellular vesicles having CD9 as an indicator.
Fig. 4 is a box plot graph of evaluation results of a plasma test specimen derived from a patient with Parkinson's disease and a plasma test specimen derived from a healthy subject using an amount of exosomes having phosphatidylserine and CD9 as an indicator.

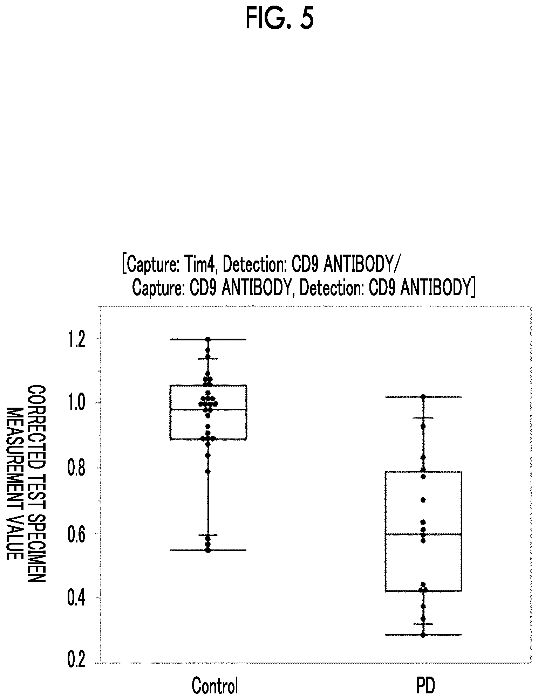

Fig. 5 is a box plot graph of evaluation results of a plasma test specimen derived from a patient with Parkinson's disease and a plasma test specimen derived from a healthy subject using a ratio of an amount of exosomes having phosphatidylserine and CD9 to an amount of extracellular vesicles having CD9 as an indicator.

### Description of Embodiments

In a case where the upper limit and the lower limit of the range are shown in the present specification, it means that A to B are A or more and B or less, unless otherwise specified.

The extracellular vesicle is a small membrane vesicle derived from a cell and composed of a lipid bilayer membrane. Examples of the extracellular vesicle include those having a diameter of 20 nm to 1,000 nm which is preferably 50 nm to 800 nm, more preferably 50 nm to 500 nm, and particularly preferably 50 nm to 200 nm. Examples of the extracellular vesicle include those classified in various ways according to the origin of its development, the size of small membrane vesicle, and the like, as described in Nature Reviews Immunology 9, 581-593 (August, 2009); "Study of Obesity", Vol. 13, No. 2, 2007, topics by Naoto Aoki et al.; and the like. Specific examples of the extracellular vesicle include an exosome, a microvesicle, an ectosome, a membrane particle, an exosome-like vesicle, an apoptotic body, and an adiposome, among which the exosome and the microvesicle are preferable, and the exosome is more preferable.

The exosome is a small membrane vesicle derived from a cell and composed of a lipid bilayer membrane, and examples thereof include those having a diameter of 50 nm to 200 nm, preferably 50 nm to 150 nm, and more preferably 50 nm to 100 nm. It should be noted that the exosome is thought to be derived from a late endosome.

The microvesicle is a small membrane vesicle derived from a cell and composed of a lipid bilayer membrane, and examples thereof include those having a diameter of 100 nm to 1,000 nm, preferably 100 nm to 800 nm, and more preferably 100 nm to 500 nm. It should be noted that the microvesicle is thought to be derived from a cell membrane.

The extracellular vesicle may be contained in a biological specimen derived from a subject or may be isolated from a biological specimen derived from a subject, and is preferably isolated from a biological specimen derived from a subject.

The biological specimen derived from a subject may be any specimen as long as it can contain extracellular vesicles, and examples thereof include a blood-derived specimen such as serum, plasma, whole blood, or buffy coat; and a body fluid specimen such as cerebrospinal fluid, urine, saliva, semen, chest exudate, tears, sputum, mucus, lymph, ascites, pleural effusion, amniotic fluid, bladder lavage fluid, or bronchial alveolar lavage fluid, among which the blood-derived specimen or the cerebrospinal fluid is preferable, the serum, the plasma, or the cerebrospinal fluid is more preferable, the serum or the plasma is still more preferable, and the plasma is particularly preferable. In addition, the blood-derived specimen is more useful from the viewpoint that the burden of sampling on the subject is light.

The biological specimen derived from a subject may be, for example, a specimen directly collected from a subject, or may be specimen that has been subjected to a pretreatment such as recovery, concentration, purification, isolation, dilution with a buffer solution or the like, or filtration sterilization. The pretreatment may be appropriately carried out according to a conventional method. Hereinafter, the biological specimen derived from a subject may be simply referred to as "biological specimen".

The method for isolating extracellular vesicles from the biological specimen may be carried out according to a conventional method, and is not particularly limited. Examples of the method for isolating extracellular vesicles from the biological specimen include an affinity method (for example, a PS affinity method), a differential centrifugation method (for example, an ultracentrifugation method such as a pellet down method, a sucrose cushion method, or a density gradient centrifugation method), an immunoprecipitation method, a chromatography method (for example, an ion exchange chromatography method or a gel permeation chromatography method), a density gradient method (for example, a sucrose density gradient method), an electrophoresis method (for example, an organelle electrophoresis method), a magnetic separation method (for example, a magnetically activated cell sorting (MACS) method), an ultrafiltration concentration method (for example, a nanomembrane ultrafiltration concentration method), a Percoll gradient isolation method, a method using a microfluidic device, and a PEG precipitation method. The affinity method is preferable from the viewpoint that extracellular vesicles having a high degree of purification can be obtained, or the differential centrifugation method is preferable from the viewpoint that it is theoretically possible to recover extracellular vesicles without bias; the affinity method or the ultracentrifugation method is more preferable; and the affinity method is particularly preferable. The PS affinity method, which is an affinity purification for phosphatidylserine, is preferable among the affinity methods. The affinity method and the differential centrifugation method may be carried out according to, for example, the method described in WO2016/088689A.

These isolation methods may be used alone or in combination of two or more thereof. In addition, isolation by one isolation method may be repeated twice or more.

The subject is not particularly limited, and examples thereof include a person diagnosed with Parkinson's disease based on the diagnostic criteria, a person diagnosed at risk of developing Parkinson's disease based on the diagnostic criteria, a person who has not been diagnosed with Parkinson's disease, a person who has not been diagnosed with Parkinson's disease based on the diagnostic criteria, and a person who has not been diagnosed at risk of developing Parkinson's disease based on the diagnostic criteria, among which the person diagnosed with Parkinson's disease based on the diagnostic criteria, the person diagnosed at risk of developing Parkinson's disease based on the diagnostic criteria, or the person who has not been diagnosed with Parkinson's disease is preferable.

Examples of the diagnostic criteria include diagnostic criteria used in a case of diagnosing Parkinson's disease based on the results of a medical interview, a test on a biomarker or the like of Parkinson's disease recommended in the Parkinson's disease clinical practice guideline such as MIBG myocardial scintigraphy or dopamine transporter scintigraphy, a test on candidate substances for the biomarker of Parkinson's disease, and the like.

The present invention relates to a method for assisting the diagnosis of Parkinson's disease, a biomarker, a reagent kit, and a device.

The present invention contains a case of determining that a subject has Parkinson's disease using a biomarker containing extracellular vesicles having phosphatidylserine and tetraspanin, and using the amount of extracellular vesicles having phosphatidylserine and tetraspanin as an indicator (hereinafter, often referred to simply as "first invention"); and a case of determining that a subject has Parkinson's disease using biomarkers containing extracellular vesicles having phosphatidylserine and tetraspanin and extracellular vesicles having tetraspanin in combination and using the ratio of an amount of extracellular vesicles having phosphatidylserine and tetraspanin to an amount of extracellular vesicles having tetraspanin as an indicator (hereinafter, often referred to simply as "second invention").

### 1. First invention

### Biomarker for assisting diagnosis of Parkinson's disease

The biomarker for assisting the diagnosis of Parkinson's disease in the first invention (hereinafter, often referred to simply as "PD marker") contains an extracellular vesicle having phosphatidylserine and tetraspanin.

The extracellular vesicle in the PD marker is the same as that described above, and preferred ones thereof are also the same.

The extracellular vesicle having phosphatidylserine and tetraspanin in the PD marker has at least one tetraspanin such as CD9, CD63, CD81, and CD151 and phosphatidylserine which is a phospholipid on the membrane surface, preferably at least one tetraspanin selected from CD9, CD63, and CD81 and phosphatidylserine, more preferably at least one tetraspanin selected from CD9 and CD81 and phosphatidylserine, and particularly preferably CD9 and phosphatidylserine.

### Method for assisting diagnosis of Parkinson's disease

The method for assisting the diagnosis of Parkinson's disease in the first invention (hereinafter, often referred to simply as "PD diagnosis assisting method") contains measuring an amount of extracellular vesicles having phosphatidylserine and tetraspanin in a biological specimen, and determining that a subject has Parkinson's disease using the amount of extracellular vesicles having phosphatidylserine and tetraspanin in the biological specimen as an indicator.

The biological specimen, the subject, and the extracellular vesicle in the PD diagnosis assisting method are the same as those described above, and preferred ones thereof are also the same.

The extracellular vesicle having phosphatidylserine and tetraspanin in the PD diagnosis assisting method is the same as that described above in the PD marker, and preferred ones thereof are also the same.

Examples of the "amount" in the PD diagnosis assisting method include mass and concentration. In addition, the "amount" also contains an actually measured value having a correlation with mass or concentration (for example, an absorbance, an amount of change in absorbance, transmitted light, an amount of change in transmitted light, a fluorescence intensity, an amount of change in fluorescence intensity, an amount of luminescence, an amount of change in amount of luminescence, a turbidity, a rate of change in turbidity, scattered light, a rate of change in scattered light, a reflectivity, an amount of change in reflectivity, a refractive index, or an amount of change in refractive index).

The measurement of the amount of extracellular vesicles having phosphatidylserine and tetraspanin in the PD diagnosis assisting method may be carried out by measuring an amount of only one type of extracellular vesicles having tetraspanin and phosphatidylserine (for example, only extracellular vesicles having CD9 and phosphatidylserine) or by measuring an amount of two or more types of extracellular vesicles having tetraspanin and phosphatidylserine (for example, extracellular vesicles having CD9 and phosphatidylserine, and extracellular vesicles having CD81 and phosphatidylserine). It is preferable to measure the amount of only one type of extracellular vesicles having tetraspanin and phosphatidylserine.

The measurement of the amount of extracellular vesicles having phosphatidylserine and tetraspanin in the PD diagnosis assisting method is not particularly limited as long as it is a method commonly carried out in this field. For example, an immunoassay using a substance having an affinity for tetraspanin and a substance having an affinity for phosphatidylserine, a mass spectrometry, a method combining these methods, or the like may be used for the measurement. Among these methods, the immunoassay using a substance having an affinity for tetraspanin and a substance having an affinity for phosphatidylserine is preferable. It should be noted that the immunoassay also contains, in addition to a method using an immune reaction (antigen-antibody reaction), a method using, for example, a binding force between two molecules other than the antigen-antibody reaction, such as binding of a lectin and a protein (method according to the immunoassay).

The substance having an affinity for tetraspanin may be any substance that specifically binds to tetraspanin, and examples thereof include a protein that specifically binds to tetraspanin, such as an antibody that specifically binds to tetraspanin or a lectin that specifically binds to a sugar chain of tetraspanin, and a nucleic acid that specifically binds to tetraspanin, among which the protein that specifically binds to tetraspanin is preferable, and the antibody that specifically binds to tetraspanin is more preferable. Examples of the antibody that specifically binds to tetraspanin include an anti-CD9 antibody, an anti-CD63 antibody, an anti-CD81 antibody, and an anti-CD151 antibody, among which the anti-CD9 antibody, the anti-CD63 antibody, or the anti-CD81 antibody is preferable, the anti-CD9 antibody or the anti-CD81 antibody is more preferable, and the anti-CD9 antibody is particularly preferable. Only one type of the substance having an affinity for tetraspanin may be used, or two or more types of the substances having an affinity for tetraspanin may be used. It is preferable to use only one type of the substance having an affinity for tetraspanin.

The substance having an affinity for phosphatidylserine may be any substance that specifically binds to phosphatidylserine, and examples thereof include a protein that specifically binds to phosphatidylserine and a nucleic acid that specifically binds to phosphatidylserine, among which the protein that specifically binds to phosphatidylserine is preferable. Examples of the protein that specifically binds to phosphatidylserine include an antibody that specifically binds to phosphatidylserine and a protein with phosphatidylserine affinity, among which the protein with phosphatidylserine affinity is preferable. Examples of the antibody that specifically binds to phosphatidylserine include an anti-phosphatidylserine antibody 1H6 (available from Merck & Co., Inc.). Examples of the protein with phosphatidylserine affinity include a Tim protein such as Tim1 (T-cell immunoglobulin-mucin domain-containing molecule 1, T-cell immunoglobulin-mucin domain 1), Tim2 (T-cell immunoglobulin-mucin domain-containing molecule 2, T-cell immunoglobulin-mucin domain 2), Tim3 (T-cell immunoglobulin-mucin domain-containing molecule 3, T-cell immunoglobulin-mucin domain 3), or Tim4 (T-cell immunoglobulin-mucin domain-containing molecule 4, T-cell immunoglobulin-mucin domain 4); an anti-phosphatidylserine antibody; Annexin V; and MFG-E8, among which the Tim protein or the anti-phosphatidylserine antibody is preferable, and the Tim protein is more preferable. In addition, the Tim protein is preferably Tim1 or Tim4 and more preferably Tim4.

Only one type of the substance having an affinity for phosphatidylserine may be used, or two or more types of the substances having an affinity for phosphatidylserine may be used. It is preferable to use only one type of the substance having an affinity for phosphatidylserine.

The substance having an affinity for tetraspanin and the substance having an affinity for phosphatidylserine may be commercially available products or substances appropriately prepared by a conventional method. In addition, the antibody that specifically binds to tetraspanin or the antibody that specifically binds to phosphatidylserine may be either a polyclonal antibody or a monoclonal antibody, and these antibodies may be used alone or in combination thereof as appropriate. In addition, not only an immunoglobulin molecule itself (intact immunoglobulin), but also a fragment antibody such as Fab, F(ab')2, or F(ab'), which is a fragment of the immunoglobulin molecule and has an ability to bind to an antigen, or a synthetic antibody such as a single chain antibody (single chain Fv), a diabody, a triabody, or a tetrabody may be used as the antibody that specifically binds to tetraspanin or the antibody that specifically binds to phosphatidylserine. In addition, in a case where these antibodies are prepared, the antibodies may be prepared, for example, according to the method described in "Immunoassay" (edited by Biochemical Assay Society of JAPAN, Kodansha Ltd., 2014).

The substance having an affinity for tetraspanin or/and the substance having an affinity for phosphatidylserine may be labeled with a labeling substance. Examples of the labeling substance include an enzyme such as peroxidase, microperoxidase, or alkaline phosphatase; a radioactive isotope such as ^{99m}Tc, ¹³¹I, ¹²⁵I, ¹⁴C, ³H, ³²P, or ³⁵S; a fluorescent substance such as fluorescein, fluorescein isothiocyanate (FITC), 4-methylumbelliferone, HiLyte, Alexa, CyDye, rhodamine, or a derivative thereof; a luminescent substance such as luciferin, luminol, or ruthenium complex; a substance having absorption in an ultraviolet region, such as phenol, naphthol, anthracene, or a derivative thereof; a substance having properties as a spin labeling agent represented by a compound having an oxyl group such as 4-amino-2,2,6,6-tetramethylpiperidine-1-oxyl; and a nanoparticle such as colloidal gold or quantum dot, among which the enzyme or the fluorescent substance is preferable. The method for labeling the substance having an affinity for tetraspanin or/and the substance having an affinity for phosphatidylserine with the labeling substance is not particularly limited, and may be carried out according to a labeling method known per se. The measurement of these labeling substances may be carried out according to a measuring method known per se depending on the labeling substance.

Using either of the substance having an affinity for tetraspanin or the substance having an affinity for phosphatidylserine as a primary affinity substance, a secondary affinity substance (for example, a secondary antibody) that specifically binds to the primary affinity substance may be further used. The secondary affinity substance may be labeled with a labeling substance and is preferably labeled with a labeling substance and more preferably a secondary antibody labeled with a labeling substance. In addition, the labeling substance and the labeling method are the same as those described above, and preferred ones thereof are also the same.

In addition, labeling with a labeling substance may be carried out taking advantage of avidin-biotin binding using the substance having an affinity for tetraspanin or/and the substance having an affinity for phosphatidylserine to which one of avidin and biotin is bound, and a labeling substance to which the other one of avidin and biotin is bound. Examples of the biotin include biotin, iminobiotin, desthiobiotin, biocytin, and biotin sulfoxide, among which the biotin is preferable. Examples of the avidin include avidin, tamavidin, tamavidin 2, and streptavidin, among which the streptavidin is preferable. The method for binding one of avidin and biotin to the substance having an affinity for tetraspanin or/and the substance having an affinity for phosphatidylserine, and the method for binding the other one of avidin and biotin to the labeling substance may be carried out according to a conventional method.

The substance having an affinity for tetraspanin or/and the substance having an affinity for phosphatidylserine may be immobilized on a solid phase, and it is preferable that the substance having an affinity for phosphatidylserine is immobilized on a solid phase.

Examples of the solid phase include synthetic polymer compounds such as latex, polystyrene, polypropylene, polyacrylic acid, polymethacrylic acid, polyacrylamide, polyglycidyl methacrylate, polyvinyl chloride, polyethylene, polychlorocarbonate, silicone resin, and silicone rubber, and inorganic substances such as porous glass, ground glass, ceramics, alumina, silica gel, activated charcoal, and metal oxide. In addition, two or more of these solid phase substances may be used in combination.

The shape of the solid phase is not particularly limited, and examples thereof include a microtiter plate (ELISA plate), a bead, a tube (microtube), a particle, a dedicated tray in which a large number of tubes are integrally molded, a disk-shaped piece, and a test tube.

The method for immobilizing the substance having an affinity for tetraspanin or/and the substance having an affinity for phosphatidylserine on a solid phase is not particularly limited as long as it is a method commonly used in this field, and may be carried out according to a conventional method.

In the PD diagnosis assisting method, the combination of the substance having an affinity for tetraspanin and the substance having an affinity for phosphatidylserine is not particularly limited, and is preferably a combination of the protein that specifically binds to tetraspanin and the protein that specifically binds to phosphatidylserine, and more preferably a combination of the antibody that specifically binds to tetraspanin and the antibody that specifically binds to phosphatidylserine or the protein with phosphatidylserine affinity.

The combination of the antibody that specifically binds to tetraspanin and the antibody that specifically binds to phosphatidylserine or the protein with phosphatidylserine affinity may be, for example, a combination of an antibody that specifically binds to tetraspanin and a Tim protein or an antibody that specifically binds to phosphatidylserine, which is preferably a combination of an anti-CD9 antibody, an anti-CD63 antibody, or an anti-CD81 antibody and a Tim protein, more preferably a combination of an anti-CD9 antibody or an anti-CD81 antibody and a Tim protein, still more preferably a combination of an anti-CD9 antibody or an anti-CD81 antibody and Tim1 or Tim4, particularly preferably a combination of an anti-CD9 antibody or an anti-CD81 antibody and Tim4, and most preferably a combination of an anti-CD9 antibody and Tim4.

Specific examples of the method for measuring the amount of extracellular vesicles having phosphatidylserine and tetraspanin include immunoassays and mass spectrometries known per se, including enzyme-linked immunosorbent assay (ELISA), enzyme immunoassay (EIA), radioimmunoassay (RIA), fluorescence enzyme immunoassay (FEIA), fluorescence immunoassay (FIA), chemiluminescence enzyme immunoassay (CLEIA), chemiluminescence immunoassay (CLIA), electrochemiluminescence immunoassay (ECLIA), immune complex transfer method, immunochromatography assay (ICA), luminescent oxygen channeling immunoassay (LOCI), capillary electrophoresis such as liquid-phase binding assay-electrokinetic analyte transport assay (LBA-EATA) and lectin electrophoresis, Western blotting, nephelometric immunoassay (NIA) such as latex nephelometric immunoassay, turbidimetric immunoassay (TIA) such as latex turbidimetric immunoassay, immunoagglutination assay such as particle counting immunoassay (PCIA), surface plasmon resonance (SPR), AlphaLISA assay, and assay for detecting the presence of a target molecule using fluorescence resonance energy transfer (FRET) or bioluminescence resonance energy transfer (BRET). Among these methods, the enzyme-linked immunosorbent assay (ELISA), the chemiluminescence enzyme immunoassay (CLEIA), the chemiluminescence immunoassay (CLIA), the electrochemiluminescence immunoassay (ECLIA), or the capillary electrophoresis is preferable; the enzyme-linked immunosorbent assay (ELISA), the chemiluminescence enzyme immunoassay (CLEIA), or LBA-EATA is more preferable; and the enzyme-linked immunosorbent assay (ELISA) is particularly preferable.

The principle for measuring the amount of extracellular vesicles having phosphatidylserine and tetraspanin is not particularly limited, and examples thereof include a sandwich method and a competitive method, among which the sandwich method is preferable. In addition, a homogeneous method, a heterogeneous method, or the like may be used as the measurement principle, and the heterogeneous method is preferable.

Examples of the method according to the immunoassay include a method of carrying out the immunoassay using the substance having an affinity for tetraspanin and the substance having an affinity for phosphatidylserine other than the antibody that specifically binds to tetraspanin and the antibody that specifically binds to phosphatidylserine, and examples of the preferred method include the same method.

The measurement of the amount of extracellular vesicles having phosphatidylserine and tetraspanin may be specifically carried out according to the method described in WO2016/088689A, the disclosure of which is incorporated by reference herein in its entirety.

The measurement of the amount of extracellular vesicles having phosphatidylserine and tetraspanin in the PD diagnosis assisting method specifically includes, for example, a method containing (1) a step of bringing extracellular vesicles having phosphatidylserine and tetraspanin in a biological specimen into contact with a substance having an affinity for tetraspanin and a substance having an affinity for phosphatidylserine to form a complex containing the extracellular vesicles having phosphatidylserine and tetraspanin in the biological specimen, the substance having an affinity for tetraspanin, and the substance having an affinity for phosphatidylserine (hereinafter, often referred to simply as "complex forming step"), and (2) a step of measuring an amount of the complex (hereinafter, often referred to simply as "complex amount measuring step") as a preferable method.

Specific examples and preferred examples of the substance having an affinity for tetraspanin, the substance having an affinity for phosphatidylserine, and a combination thereof are the same as those described above.

The order in which the substance having an affinity for tetraspanin, the substance having an affinity for phosphatidylserine, and the biological specimen are brought into contact with each other in the complex forming step is not particularly limited, and it is preferable to contact the biological specimen with the substance having an affinity for phosphatidylserine and then the substance having an affinity for tetraspanin.

That is, the complex forming step preferably contains a first procedure of bringing a biological specimen into contact with a substance having an affinity for phosphatidylserine to form a first complex composed of extracellular vesicles in the biological specimen and the substance having an affinity for phosphatidylserine, and a second procedure of bringing the first complex into contact with a substance having an affinity for tetraspanin to form a second complex composed of the first complex and the substance having an affinity for tetraspanin.

Specifically, in the complex forming step, for example, an antibody or Tim protein (preferably Tim1 or Tim4 and more preferably Tim4) that specifically binds to phosphatidylserine immobilized on a solid phase and a biological specimen are brought into contact with each other to form a first complex of the antibody or Tim protein that specifically binds to phosphatidylserine and an extracellular vesicle having phosphatidylserine and tetraspanin in the biological specimen, and the first complex and an antibody that specifically binds to tetraspanin are brought into contact with each other to form a second complex of the first complex and the antibody that specifically binds to tetraspanin.

After forming the complex, it is preferable to carry out a washing operation (B/F separation) at least before the complex amount measuring step.

Specifically, for example, the washing operation may be carried out after forming the first complex or/and after forming the second complex in the above method. It is preferable to carry out the washing operation (B/F separation) after forming the first complex, and further carry out the washing operation (B/F separation) after forming the second complex.

The complex amount measuring step is a step of measuring an amount of the complex containing extracellular vesicles having phosphatidylserine and tetraspanin, a substance having an affinity for tetraspanin, and a substance having an affinity for phosphatidylserine obtained in the complex forming step. Any method may be used as long as the amount of the complex can be measured.

More specifically, the complex amount measuring step may detect a labeling substance in the complex containing an antibody or Tim protein (preferably Tim1 or Tim4 and more preferably Tim4) that specifically binds to phosphatidylserine immobilized on a solid phase, an extracellular vesicle having phosphatidylserine and tetraspanin in a biological specimen, an antibody that specifically binds to tetraspanin, and a labeling substance (preferably an enzyme or a fluorescent substance), obtained in the complex forming step using, for example, (1) an antibody that specifically binds to tetraspanin labeled with a labeling substance, or (2) a labeled secondary antibody labeled with a labeling substance, which specifically binds to an "antibody that specifically binds to tetraspanin", or (3) an antibody that specifically binds to tetraspanin to which one of avidin and biotin is bound and a labeling substance to which the other one of avidin and biotin is bound.

In the complex amount measuring step, it is preferable to carry out a washing operation (B/F separation) before detecting a labeling substance.

More specifically, the measurement of the amount of extracellular vesicles having phosphatidylserine and tetraspanin in the PD diagnosis assisting method may be carried out, for example, in such a manner that an antibody or Tim protein (preferably Tim1 or Tim4 and more preferably Tim4) that specifically binds to phosphatidylserine immobilized on a solid phase plate and a biological specimen are brought into contact with each other to form a first complex of the antibody or Tim protein that specifically binds to phosphatidylserine and an extracellular vesicle having phosphatidylserine and tetraspanin in the biological specimen, followed by B/F separation if necessary, (1) the first complex is brought into contact with an antibody that specifically binds to tetraspanin labeled with a labeling substance to form a second complex of the first complex and the antibody that specifically binds to tetraspanin labeled with a labeling substance, (2) the first complex is brought into contact with an antibody that specifically binds to tetraspanin to form a second complex of the first complex and the antibody that specifically binds to tetraspanin, followed by B/F separation if necessary, and the second complex is brought into contact with a labeled secondary antibody labeled with a labeling substance, which specifically binds to "the antibody that specifically binds to tetraspanin", to form a third complex of the second complex and the labeled secondary antibody, or (3) the first complex is brought into contact with an antibody that specifically binds to tetraspanin to which one of avidin and biotin is bound to form a second complex of the first complex and the antibody that specifically binds to tetraspanin to which one of avidin and biotin is bound, followed by B/F separation if necessary, a third complex of the second complex and a labeling substance (preferably an enzyme or a fluorescent substance) to which the other one of avidin and biotin is bound is formed, followed by B/F separation, and then the labeling substance of the obtained second complex or third complex is detected.

The amount of the biological specimen and the amount (concentration) of protein in the biological specimen, the amount (concentration) and number of particles of extracellular vesicles having phosphatidylserine and tetraspanin in the biological specimen, and the amount (concentration) of the substance having an affinity for tetraspanin and the substance having an affinity for phosphatidylserine to react with these extracellular vesicles, the labeling substance and the labeling method, and the like may be appropriately set according to the type of biological specimen, the required measurement sensitivity, the measuring method and measuring device to be used, and the like.

In addition, the amount (concentration) of extracellular vesicles having phosphatidylserine and tetraspanin in the biological specimen may be calculated by creating a calibration curve using a reference standard. Examples of the reference standard include an extracellular vesicle having phosphatidylserine and tetraspanin.

The determination of Parkinson's disease in the PD diagnosis assisting method contains determining Parkinson's disease using the amount of extracellular vesicles having phosphatidylserine and tetraspanin as an indicator.

Examples of the determination of Parkinson's disease include determination of whether or not a subject is likely to have Parkinson's disease, determination of whether or not a subject may have Parkinson's disease, determination of whether or not a subject is at high risk of developing Parkinson's disease, and determination of whether or not a subject is at risk of developing Parkinson's disease, among which the determination of whether or not a subject is likely to have Parkinson's disease or the determination of whether or not a subject may have Parkinson's disease is preferable.

The determination of Parkinson's disease in the PD diagnosis assisting method is made, for example, by comparing the amount of extracellular vesicles having phosphatidylserine and tetraspanin in a biological specimen derived from a subject, obtained by measuring the amount of extracellular vesicles having phosphatidylserine and tetraspanin, with a predetermined reference value (cutoff value).

Specifically, in a case where the amount of extracellular vesicles having phosphatidylserine and tetraspanin is equal to or less than a predetermined reference value, it can be determined that a subject is likely to have Parkinson's disease; or a subject may have Parkinson's disease; or a subject is at high risk of developing Parkinson's disease; or a subject is at risk of developing Parkinson's disease.

In addition, in a case where the amount of extracellular vesicles having phosphatidylserine and tetraspanin is larger than a predetermined reference value, it can be determined that a subject is unlikely to have Parkinson's disease; or a subject may not have Parkinson's disease; or a subject is at low risk of developing Parkinson's disease; or a subject is not at risk of developing Parkinson's disease.

The predetermined reference value (cutoff value) is an amount of extracellular vesicles having phosphatidylserine and tetraspanin in a biological specimen, which is preset to distinguish between a patient with Parkinson's disease and a healthy subject.

The method for determining the predetermined reference value is not particularly limited. For example, the predetermined reference value can be determined in such a manner that an amount of extracellular vesicles having phosphatidylserine and tetraspanin contained in a biological specimen obtained from a patient suffering from Parkinson's disease and a healthy subject is measured, and a statistical analysis such as receiver operating characteristic analysis (ROC analysis) is carried out using the obtained amount of extracellular vesicles having phosphatidylserine and tetraspanin. In setting the predetermined reference value, it is preferable to consider sensitivity, specificity, positive predictive value, negative predictive value, and the like. For example, the predetermined reference value can be set such that the sensitivity is 60% or more, preferably 70% or more, more preferably 80% or more, and still more preferably 90% or more. For example, the predetermined reference value can be set such that the specificity is 60% or more, preferably 70% or more, more preferably 80% or more, and still more preferably 90% or more.

Use of a reagent **kit for assisting diagnosis of Parkinson's disease**

The reagent kit for assisting the diagnosis of Parkinson's disease in the first invention (hereinafter, often referred to simply as "reagent kit for assisting the diagnosis of PD") contains a substance having an affinity for tetraspanin and a substance having an affinity for phosphatidylserine.

The substance having an affinity for tetraspanin and the substance having an affinity for phosphatidylserine in the reagent kit for assisting the diagnosis of PD are the same as those described above in the PD diagnosis assisting method, and preferred ones thereof are also the same.

The substance having an affinity for tetraspanin and the substance having an affinity for phosphatidylserine each may be in a solution state, a frozen state, a dried state, or a freeze-dried state. In addition, the substance having an affinity for tetraspanin and the substance having an affinity for phosphatidylserine may be contained in the kit as one reagent or may be contained in the kit as separate reagents.

The combination of the substance having an affinity for tetraspanin and the substance having an affinity for phosphatidylserine in the reagent kit for assisting the diagnosis of PD includes the same combinations as those described above in the PD diagnosis assisting method, and preferred combinations thereof are also the same.

The substance having an affinity for tetraspanin or/and the substance having an affinity for phosphatidylserine in the reagent kit for assisting the diagnosis of PD may be immobilized on a solid phase and may be labeled with a labeling substance. The solid phase, the method for immobilizing the substance on the solid phase, the labeling substance, and the labeling method are the same as those described above in the PD diagnosis assisting method, and preferred ones thereof are also the same.

The reagent kit for assisting the diagnosis of PD may further contain a secondary affinity substance (for example, a secondary antibody) that specifically binds to the substance having an affinity for tetraspanin or/and the substance having an affinity for phosphatidylserine. The secondary affinity substance in the reagent kit for assisting the diagnosis of PD is the same as that described above in the PD diagnosis assisting method, and preferred ones thereof are also the same. In addition, the secondary affinity substance in the reagent kit for assisting the diagnosis of PD may be labeled with a labeling substance. The labeling substance and the labeling method are the same as those described above in the PD diagnosis assisting method and preferred ones thereof are also the same.

The substance having an affinity for tetraspanin or/and the substance having an affinity for phosphatidylserine in the reagent kit for assisting the diagnosis of PD may be a substance to which one of avidin and biotin is bound. The avidin and the biotin are the same as those described above in the PD diagnosis assisting method, and preferred ones thereof are also the same.

The reagent kit for assisting the diagnosis of PD may further contain a labeling substance to which one of avidin and biotin is bound. The labeling substance and labeling method are the same as those described above in the PD diagnosis assisting method, and preferred ones thereof are also the same.

The concentration (amount) of the substance having an affinity for tetraspanin and the substance having an affinity for phosphatidylserine in the reagent kit for assisting the diagnosis of PD may be appropriately set within the range commonly used in this field depending on the measuring method. For example, the substance having an affinity for tetraspanin is contained at a concentration at the time of use of usually 10 to 20,000 ng/mL and preferably 100 to 10,000 ng/mL, in a case of being immobilized on a solid phase, and at a concentration of usually 10 to 5,000 ng/mL and preferably 100 to 500 ng/mL in a case of being used for detection. In addition, for example, the substance having an affinity for phosphatidylserine is contained at a concentration at the time of use of usually 10 to 20,000 ng/mL and preferably 100 to 10,000 ng/mL, in a case of being immobilized on a solid phase, and at a concentration of usually 10 to 5,000 ng/mL and preferably 100 to 500 ng/mL in a case of being used for detection.

In addition, reagents commonly used in this field, for example, a buffer, a reaction promoter, a sugar, a protein, a salt, a stabilizer such as surfactant, and a preservative, may coexist with the substance having an affinity for tetraspanin or/and the substance having an affinity for phosphatidylserine. The concentration and pH of these reagents may be appropriately selected from the ranges commonly used in this field.

Further, the reagent kit for assisting the diagnosis of PD may contain, in addition to the substance having an affinity for tetraspanin and the substance having an affinity for phosphatidylserine, a reagent needed to measure an amount of extracellular vesicles having tetraspanin and phosphatidylserine using these affinity substances. Examples of such a reagent include a washing agent, a specimen diluent (a reagent for diluting a specimen), a reagent for detecting a labeling substance, a reagent for binding a labeling substance to these affinity substances, a reagent for immobilizing these affinity substances on a solid phase, and a reagent for binding avidin or biotin to these affinity substances and labeling substances. The concentration, pH, and the like of these reagents may be appropriately selected from the ranges commonly used in this field.

The reagent kit for assisting the diagnosis of PD may contain a reference standard used to create a calibration curve for extracellular vesicles having phosphatidylserine and tetraspanin. Examples of the reference standard include an extracellular vesicle having phosphatidylserine and tetraspanin. The reference standard may be in a solution state, a frozen state, a dried state, or a freeze-dried state. In addition, reagents commonly used in this field, for example, a buffer, a reaction promoter, a sugar, a protein, a salt, a stabilizer such as a surfactant, and a preservative, may coexist with the reference standard. The concentration and pH of these reagents may be appropriately selected from the ranges commonly used in this field.

The reagent kit for assisting the diagnosis of PD may contain a package insert or an instruction manual. Examples of the package insert or the instruction manual include a package insert or instruction manual stating that a subject is determined to have Parkinson's disease by measuring an amount of extracellular vesicles having phosphatidylserine and tetraspanin in a biological specimen derived from a subject or/and using the amount of extracellular vesicles having phosphatidylserine and tetraspanin as an indicator. These package insert and instruction manual may be described separately in a plurality of cases, or may be described collectively in one.

Specifically, the reagent kit for assisting the diagnosis of PD may be, for example, a kit containing one of a substance having an affinity for tetraspanin and a substance having an affinity for phosphatidylserine immobilized on a solid phase and the other one of the substance having an affinity for tetraspanin and the substance having an affinity for phosphatidylserine bound to a labeling substance or the other one affinity substance and a component for indirectly binding the other one affinity substance to a labeling substance.

The reagent kit for assisting the diagnosis of PD is preferably, for example, a kit containing either one of the substance having an affinity for tetraspanin and the substance having an affinity for phosphatidylserine immobilized on a solid phase and any one selected from the following (1) to (3).

(1) the other one of the substance having an affinity for tetraspanin and the substance having an affinity for phosphatidylserine bound to a labeling substance, (2) the other one of the substance having an affinity for tetraspanin and the substance having an affinity for phosphatidylserine, and a secondary affinity substance that specifically binds to the affinity substance bound to a labeling substance, and (3) the other one of the substance having an affinity for tetraspanin and the substance having an affinity for phosphatidylserine to which one of avidin and biotin is bound, and a labeling substance to which the other one of avidin and biotin is bound.

Preferred specific examples of the reagent kit for assisting the diagnosis of PD include a kit containing a solid phase plate on which an antibody or Tim protein (more preferably Tim4 or Tim1 and particularly preferably the Tim4) that specifically binds to phosphatidylserine is immobilized, and any one selected from the following (1) to (3).

(1) a solution containing the other one of the antibody (more preferably an anti-CD9 antibody or an anti-CD81 antibody and particularly preferably the anti-CD9 antibody) or Tim protein that specifically binds to phosphatidylserine bound to a labeling substance (usually 10 to 5,000 ng/mL and preferably 100 to 500 ng/mL), (2) a solution containing the other one of the antibody or Tim protein that specifically binds to phosphatidylserine (usually 10 to 5,000 ng/mL and preferably 100 to 500 ng/mL), and a solution containing a secondary affinity substance that specifically binds to the affinity substance bound to a labeling substance (usually 10 to 5,000 ng/mL and preferably 100 to 500 ng/mL), and (3) a solution containing an antibody (preferably an anti-CD9 antibody or an anti-CD81 antibody and more preferably the anti-CD9 antibody) that specifically binds to tetraspanin to which one of avidin and biotin is bound (usually 10 to 5,000 ng/mL and preferably 100 to 500 ng/mL), and a solution containing a labeling substance to which the other one of avidin and biotin is bound (usually 10 to 5,000 ng/mL and preferably 100 to 500 ng/mL).

### Device for assisting diagnosis of Parkinson's disease (falling outside the scope of the claims)

The device for assisting the diagnosis of Parkinson's disease in the first invention (hereinafter, often referred to simply as "device for assisting the diagnosis of PD") has a measurement unit that measures an amount of extracellular vesicles having phosphatidylserine and tetraspanin in a biological specimen derived from a subject, and a determination unit that determines that the subject has Parkinson's disease using the amount of extracellular vesicles having phosphatidylserine and tetraspanin in the biological specimen as an indicator.

The biological specimen, the subject, and the extracellular vesicle in the device for assisting the diagnosis of PD are the same as those described above, and preferred ones thereof are also the same.

The measurement unit, the determination unit, and the like constituting the device for assisting the diagnosis of PD may be arranged in the same device or may be separate bodies.

The measurement unit in the device for assisting the diagnosis of PD is a site for measuring the amount of extracellular vesicles having phosphatidylserine and tetraspanin in the biological specimen introduced into the device. The size and configuration of the measurement unit are not particularly limited. Examples of the measurement unit include an imaging apparatus for imaging using a microplate reader or CCD used for ELISA, an imaging apparatus used for a method using Western blotting or a microarray (microchip), a mass spectrometer used for mass spectrometry, an intermolecular interaction analyzer, a flow cytometer used for flow cytometry, and an ultraviolet/visible light detector or fluorescence detector used for HPLC or capillary electrophoresis.

The extracellular vesicle having phosphatidylserine and tetraspanin, the amount, and the measurement target of the method for measuring the amount of extracellular vesicles having phosphatidylserine and tetraspanin in the device for assisting the diagnosis of PD are the same as those described above in the PD diagnosis assisting method, and preferred ones thereof are also the same.

The measurement of extracellular vesicles having phosphatidylserine and tetraspanin and the substances used for the measurement in the device for assisting the diagnosis of PD are the same as those described above in the PD diagnosis assisting method, and preferred ones and specific examples thereof are also the same.

The device for assisting the diagnosis of PD may contain a calculation unit. The calculation unit in the device for assisting the diagnosis of PD is a site for converting an actually measured value (for example, an absorbance, an amount of change in absorbance, transmitted light, an amount of change in transmitted light, a fluorescence intensity, an amount of change in fluorescence intensity, an amount of luminescence, an amount of change in amount of luminescence, a turbidity, a rate of change in turbidity, scattered light, a rate of change in scattered light, a reflectivity, an amount of change in reflectivity, a refractive index, or an amount of change in refractive index) having a correlation with the mass or concentration of extracellular vesicles having phosphatidylserine and tetraspanin in the biological specimen obtained by the measurement unit into mass, concentration, or the like.

It should be noted that the actually measured value, and the mass, concentration, or the like converted by the calculation unit may be stored in a storage device or the like provided in a device such as a memory device or a hard disk.

The determination unit in the device for assisting the diagnosis of PD is a site for determining Parkinson's disease using the amount of extracellular vesicles having phosphatidylserine and tetraspanin obtained by the measurement unit or the calculation unit as an indicator.

The determination of Parkinson's disease and the predetermined reference value used for the determination in the device for assisting the diagnosis of PD, and the method for determining the predetermined reference value are the same as those described above in the PD diagnosis assisting method and preferred ones and specific examples thereof are also the same.

The predetermined reference value in the device for assisting the diagnosis of PD may be stored in advance in the device for assisting the diagnosis of PD, or may be input from an input site of the device for assisting the diagnosis of PD at the time of determination.

The device for assisting the diagnosis of PD may contain an output unit. The output unit carries out processing such as displaying or outputting the results of determination to a display device such as a display or a printing device such as a printer.

### 2. Second invention

### Biomarker set for assisting diagnosis of Parkinson's disease

The biomarker set for assisting the diagnosis of Parkinson's disease in the second invention (hereinafter, often referred to simply as "PD marker set") is a combination of biomarkers containing extracellular vesicles having phosphatidylserine and tetraspanin and extracellular vesicles having tetraspanin.

According to the PD marker set, for example, a ratio of the amount of extracellular vesicles having phosphatidylserine and tetraspanin to the amount of extracellular vesicles having tetraspanin (hereinafter, often referred to simply as "PD marker (II)") can be obtained, and the ratio can be used as an indicator for determining that a subject has Parkinson's disease.

In addition, according to the PD marker (II), it can be used as an indicator for distinguishing whether the subject has Parkinson's disease with cognitive symptoms or Parkinson's disease without cognitive symptoms.

The extracellular vesicle in the PD marker set is the same as the extracellular vesicle described above, and preferred ones thereof are also the same.

The extracellular vesicle having phosphatidylserine and tetraspanin in the PD marker set has at least one tetraspanin such as CD9, CD63, CD81, and CD151 and phosphatidylserine which is a phospholipid on the membrane surface, preferably at least one tetraspanin selected from CD9, CD63, and CD81 and phosphatidylserine, more preferably at least one tetraspanin selected from CD9 and CD63 and phosphatidylserine, and particularly preferably CD9 and phosphatidylserine.

The extracellular vesicle having tetraspanin in the PD marker set has at least one tetraspanin such as CD9, CD63, CD81, and CD151 on the membrane surface and preferably has at least one tetraspanin selected from CD9, CD63, and CD81, more preferably at least one tetraspanin selected from CD9 and CD63, and particularly preferably CD9.

The tetraspanin in the extracellular vesicle having phosphatidylserine and tetraspanin in the PD marker set may be the same as or different from the tetraspanin in the extracellular vesicle having tetraspanin, and is preferably the same as the tetraspanin in the extracellular vesicle having tetraspanin.

### Method (II) of assisting diagnosis of Parkinson's disease

The method for assisting the diagnosis of Parkinson's disease in the second invention (hereinafter, often referred to simply as "PD diagnosis assisting method (II)") contains measuring an amount of extracellular vesicles having phosphatidylserine and tetraspanin in a biological specimen and an amount of extracellular vesicles having tetraspanin in the biological specimen; calculating a ratio of the amount of extracellular vesicles having phosphatidylserine and tetraspanin to the amount of extracellular vesicles having tetraspanin (PD marker (II)); and determining that a subject has Parkinson's disease using the ratio of the amount of extracellular vesicles having phosphatidylserine and tetraspanin to the amount of extracellular vesicles having tetraspanin as an indicator.

The biological specimen, the subject, and the extracellular vesicle in the PD diagnosis assisting method (II) are the same as those described above, and preferred ones thereof are also the same.

The extracellular vesicle having phosphatidylserine and tetraspanin in the PD diagnosis assisting method (II) is the same as that described above in the PD marker set, and preferred ones thereof are also the same.

The amount, the target for measuring the amount of extracellular vesicles having phosphatidylserine and tetraspanin, and the measurement of the amount of extracellular vesicles having phosphatidylserine and tetraspanin and the substances used for the measurement in the PD diagnosis assisting method (II) are the same as those described above in the PD diagnosis assisting method, and preferred ones and specific examples thereof except for an antibody that specifically binds to tetraspanin, and a combination of an antibody that specifically binds to tetraspanin and an antibody that specifically binds to phosphatidylserine or a protein with phosphatidylserine affinity are also the same as those described above in the PD diagnosis assisting method.

Examples of the antibody that specifically binds to tetraspanin in the PD diagnosis assisting method (II) include an anti-CD9 antibody, an anti-CD63 antibody, an anti-CD81 antibody, and an anti-CD151 antibody, among which the anti-CD9 antibody, the anti-CD63 antibody, or the anti-CD81 antibody is preferable, the anti-CD9 antibody or the anti-CD63 antibody is more preferable, and the anti-CD9 antibody is particularly preferable.

Only one type of the substance having an affinity for tetraspanin may be used, or two or more types of the substances having an affinity for tetraspanin may be used. It is preferable to use only one type of the substance having an affinity for tetraspanin.

The combination of the antibody that specifically binds to tetraspanin and the antibody that specifically binds to phosphatidylserine or the protein with phosphatidylserine affinity in the PD diagnosis assisting method (II) may be, for example, a combination of an antibody that specifically binds to tetraspanin and a Tim protein or an antibody that specifically binds to phosphatidylserine, which is preferably a combination of an anti-CD9 antibody, an anti-CD63 antibody, or an anti-CD81 antibody and a Tim protein, more preferably a combination of an anti-CD9 antibody or an anti-CD63 antibody and a Tim protein, still more preferably a combination of an anti-CD9 antibody or an anti-CD63 antibody and Tim1 or Tim4, particularly preferably a combination of an anti-CD9 antibody or an anti-CD63 antibody and Tim4, and most preferably a combination of an anti-CD9 antibody and Tim4.

The extracellular vesicle having tetraspanin in the PD diagnosis assisting method (II) is the same as that described above in the PD marker set, and preferred ones thereof are also the same.

The tetraspanin in the extracellular vesicle having phosphatidylserine and tetraspanin in the PD diagnosis assisting method (II) may be the same as or different from the tetraspanin in the extracellular vesicle having tetraspanin, and is preferably the same as the tetraspanin in the extracellular vesicle having tetraspanin.

The measurement of the amount of extracellular vesicles having tetraspanin in the PD diagnosis assisting method (II) may be carried out by measuring an amount of one type of extracellular vesicles having tetraspanin (for example, only extracellular vesicles having CD9) or by measuring an amount of two or more types of extracellular vesicles having tetraspanin (for example, extracellular vesicles having CD9 and extracellular vesicles having CD63). It is preferable to measure the amount of only one type of extracellular vesicles having tetraspanin.

The method for measuring the amount of extracellular vesicles having tetraspanin is not particularly limited as long as it is a method commonly used in this field, and examples thereof include an immunoassay using a substance having an affinity for tetraspanin, a mass spectrometry, and a method combining these methods, among which the immunoassay using a substance having an affinity for tetraspanin is preferable. It should be noted that the immunoassay contains not only a method using an immune reaction (antigen-antibody reaction) but also a method using, for example, a binding force between two molecules other than the antigen-antibody reaction, such as binding of a lectin and a protein (method according to the immunoassay).

Examples of the substance having an affinity for tetraspanin in the measurement of the amount of extracellular vesicles having tetraspanin include the same substances as those described above in the PD diagnosis assisting method, among which a protein that specifically binds to tetraspanin is preferable, and an antibody that specifically binds to tetraspanin is more preferable. Examples of the antibody that specifically binds to tetraspanin include an anti-CD9 antibody, an anti-CD63 antibody, an anti-CD81 antibody, and an anti-CD151 antibody, among which the anti-CD9 antibody or the anti-CD63 antibody is preferable, and the anti-CD9 antibody is more preferable. Only one type of the substance having an affinity for tetraspanin may be used, or two or more types of the substances having an affinity for tetraspanin may be used. It is preferable to use only one type of the substance having an affinity for tetraspanin.

The substance having an affinity for tetraspanin may be a commercially available product or a substance appropriately prepared by a conventional method. In addition, the antibody that specifically binds to tetraspanin may be either a polyclonal antibody or a monoclonal antibody, and these antibodies may be used alone or in combination thereof as appropriate. In addition, not only an immunoglobulin molecule itself (intact immunoglobulin), but also a fragment antibody such as Fab, F(ab')2, or F(ab'), which is a fragment of the immunoglobulin molecule and has an ability to bind to an antigen, or a synthetic antibody such as a single chain antibody (single chain Fv), a diabody, a triabody, or a tetrabody may be used as the antibody that specifically binds to tetraspanin. In addition, in a case where these antibodies are prepared, the antibodies may be prepared, for example, according to the method described in "Immunoassay" (edited by Biochemical Assay Society of JAPAN, Kodansha Ltd., 2014).

The substance having an affinity for tetraspanin in the PD diagnosis assisting method (II) may be labeled with a labeling substance. The labeling substance and the labeling method are the same as those of the PD diagnosis assisting method and preferred ones and specific examples thereof are also the same.

In addition, in the same manner as in the PD diagnosis assisting method, using the substance having an affinity for tetraspanin as a primary affinity substance, a secondary affinity substance (for example, a secondary antibody) that specifically binds to the primary affinity substance may be further used. The secondary affinity substance may be labeled with a labeling substance. The labeling substance and the labeling method are the same as those described above in the PD diagnosis assisting method and preferred ones thereof are also the same.

Further, labeling with a labeling substance may be carried out taking advantage of avidin-biotin binding using the substance having an affinity for tetraspanin to which one of avidin and biotin is bound, and a labeling substance to which the other one of avidin and biotin is bound. The avidin, the biotin, and the method for binding avidin or biotin to the substance having an affinity for tetraspanin are the same as those described above in the PD diagnosis assisting method, and preferred ones and specific examples thereof are also the same.

The substance having an affinity for tetraspanin in the PD diagnosis assisting method (II) may be immobilized on a solid phase. The solid phase and the method for immobilizing the substance having an affinity for tetraspanin on the solid phase are the same as those described above in the PD diagnosis assisting method, and preferred ones and specific examples thereof are also the same.

With regard to the substance having an affinity for tetraspanin used for measuring extracellular vesicles having phosphatidylserine and tetraspanin and the substance having an affinity for tetraspanin used for measuring extracellular vesicles having tetraspanin in the PD diagnosis assisting method (II), at least one same substance having an affinity for tetraspanin may be used (for example, at least a substance having an affinity for CD9 is used for the measurement of both), or different types of substances having an affinity for tetraspanin may be used (for example, a substance having an affinity for CD9 is used for the measurement of one, and a substance having an affinity for CD63 is used for the measurement of the other one). It is preferable to use at least one same substance having an affinity for tetraspanin; it is more preferable to use only a substance having an affinity for the same tetraspanin (for example, only a substance having an affinity for CD9 is used for the measurement of both); and it is particularly preferable to use only one type of substance having an affinity for the same tetraspanin (for example, only an anti-CD9 antibody is used for the measurement of both).

The measurement and measurement principle of the amount of extracellular vesicles having tetraspanin in the PD diagnosis assisting method (II) are the same as those described above as the measurement and measurement principle of the amount of extracellular vesicles having phosphatidylserine and tetraspanin in the PD diagnosis assisting method, and preferred ones thereof are also the same.

The measurement of the amount of extracellular vesicles having tetraspanin in the PD diagnosis assisting method (II) specifically includes, for example, a method containing (1) a step of bringing extracellular vesicles having tetraspanin in a biological specimen into contact with a substance having an affinity for tetraspanin to form a complex containing the extracellular vesicles having tetraspanin in the biological specimen and the substance having an affinity for tetraspanin (complex forming step), and (2) a step of measuring an amount of the complex (complex amount measuring step) as a preferable method.

The complex forming step in the measurement of the amount of extracellular vesicles having tetraspanin in the PD diagnosis assisting method (II) preferably contains a first procedure of bringing a biological specimen into contact with a substance having an affinity for tetraspanin to form a first complex composed of extracellular vesicles in the biological specimen and the substance having an affinity for tetraspanin, and a second procedure of bringing the first complex into contact with a substance having an affinity for tetraspanin to form a second complex composed of the first complex and the substance having an affinity for tetraspanin.

It is preferable that the substance having an affinity for tetraspanin used for forming the first complex and the substance having an affinity for tetraspanin used for forming the second complex are the same.

The complex forming step in the measurement of the amount of extracellular vesicles having tetraspanin in the PD diagnosis assisting method (II) is specifically carried out, for example, in such a manner that an antibody that specifically binds to tetraspanin (preferably an anti-CD9 antibody or an anti-CD63 antibody and more preferably the anti-CD9 antibody) immobilized on a solid phase is brought into contact with a biological specimen to form a first complex of the antibody that specifically binds to tetraspanin and extracellular vesicles having phosphatidylserine and tetraspanin in the biological specimen, and the first complex is brought into contact with an antibody that specifically binds to tetraspanin (preferably an anti-CD9 antibody or an anti-CD63 antibody and more preferably the anti-CD9 antibody) to form a second complex of the first complex and the antibody that specifically binds to tetraspanin.

After forming the complex, it is preferable to carry out a washing operation (B/F separation) at least before the complex amount measuring step.

Specifically, for example, the washing operation may be carried out after forming the first complex or/and after forming the second complex in the above method. It is preferable to carry out the washing operation (B/F separation) after forming the first complex, and further carry out the washing operation (B/F separation) after forming the second complex.

The complex amount measuring step in the measurement of the amount of extracellular vesicles having tetraspanin in the PD diagnosis assisting method (II) is a step of measuring an amount of the complex containing extracellular vesicles having tetraspanin and a substance having an affinity for tetraspanin obtained in the complex forming step. Any method may be used as long as the amount of the complex can be measured.

More specifically, the complex amount measuring step may detect a labeling substance in the complex containing an antibody that specifically binds to tetraspanin (preferably an anti-CD9 antibody or an anti-CD63 antibody and more preferably the anti-CD9 antibody) immobilized on a solid phase plate, an extracellular vesicle having tetraspanin in a biological specimen, an antibody that specifically binds to tetraspanin (preferably an anti-CD9 antibody or an anti-CD63 antibody and more preferably the anti-CD9 antibody), and a labeling substance (preferably an enzyme or a fluorescent substance) obtained in the complex forming step using, for example, (1) an antibody that specifically binds to tetraspanin labeled with a labeling substance, or (2) a labeled secondary antibody labeled with a labeling substance, which specifically binds to an "antibody that specifically binds to tetraspanin", or (3) an antibody that specifically binds to tetraspanin to which one of avidin and biotin is bound and a labeling substance to which the other one of avidin and biotin is bound.

In the complex amount measuring step, it is preferable to carry out a washing operation (B/F separation) before detecting a labeling substance.

More specifically, the measurement of the amount of extracellular vesicles having tetraspanin in the PD diagnosis assisting method (II) may be carried out, for example, in such a manner that an antibody that specifically binds to tetraspanin (preferably an anti-CD9 antibody or an anti-CD63 antibody and more preferably the anti-CD9 antibody) immobilized on a solid phase plate and a biological specimen are brought into contact with each other to form a first complex of the antibody that specifically binds to tetraspanin and an extracellular vesicle having tetraspanin in the biological specimen, followed by B/F separation if necessary, (1) the first complex is brought into contact with an antibody that specifically binds to tetraspanin (preferably an anti-CD9 antibody or an anti-CD63 antibody and more preferably the anti-CD9 antibody) labeled with a labeling substance to form a second complex of the first complex and the antibody that specifically binds to tetraspanin labeled with a labeling substance, (2) the first complex is brought into contact with an antibody that specifically binds to tetraspanin (preferably an anti-CD9 antibody or an anti-CD63 antibody and more preferably the anti-CD9 antibody) to form a second complex of the first complex and the antibody that specifically binds to tetraspanin, followed by B/F separation if necessary, and the second complex is brought into contact with a labeled secondary antibody labeled with a labeling substance, which specifically binds to "the antibody that specifically binds to tetraspanin", to form a third complex of the second complex and the labeled secondary antibody, or (3) the first complex is brought into contact with an antibody that specifically binds to tetraspanin (preferably an anti-CD9 antibody or an anti-CD63 antibody and more preferably the anti-CD9 antibody) to which one of avidin and biotin is bound to form a second complex of the first complex and the antibody that specifically binds to tetraspanin to which one of avidin and biotin is bound, followed by B/F separation if necessary, a third complex of the second complex and a labeling substance (preferably an enzyme or a fluorescent substance) to which the other one of avidin and biotin is bound is formed, followed by B/F separation, and then the labeling substance (preferably an enzyme or a fluorescent substance) of the obtained second complex or third complex is detected.

The amount of the biological specimen and the amount (concentration) of protein in the biological specimen, the amount (concentration) and number of particles of extracellular vesicles having tetraspanin in the biological specimen, the amount (concentration) of the substance having an affinity for tetraspanin to react with these extracellular vesicles, the labeling substance and the labeling method, and the like may be appropriately set according to the type of biological specimen, the required measurement sensitivity, the measuring method and measuring device to be used, and the like.

In addition, the amount (concentration) of extracellular vesicles having tetraspanin in the biological specimen may be calculated by creating a calibration curve using a reference standard. Examples of the reference standard include an extracellular vesicle having tetraspanin.

The ratio of the amount of extracellular vesicles having phosphatidylserine and tetraspanin to the amount of extracellular vesicles having tetraspanin may be calculated by dividing the amount (A) of extracellular vesicles having phosphatidylserine and tetraspanin by the amount (B) of extracellular vesicles having tetraspanin ((A)/(B)).

The determination of Parkinson's disease in the PD diagnosis assisting method (II) contains determining Parkinson's disease using the ratio ((A)/(B)) of the amount (B) of extracellular vesicles having phosphatidylserine and tetraspanin to the amount (A) of extracellular vesicles having tetraspanin as an indicator.

Examples of the determination of Parkinson's disease include determination of whether or not a subject is likely to have Parkinson's disease, determination of whether or not a subject may have Parkinson's disease, determination of whether or not a subject is at high risk of developing Parkinson's disease, and determination of whether or not a subject is at risk of developing Parkinson's disease, among which the determination of whether or not a subject is likely to have Parkinson's disease or the determination of whether or not a subject may have Parkinson's disease is preferable.

In addition, the determination of Parkinson's disease in the PD diagnosis assisting method (II) may contain determining (distinguishing) whether the subject has Parkinson's disease with cognitive symptoms or Parkinson's disease without cognitive symptoms, using the ratio ((A)/(B)) of the amount (B) of the extracellular vesicles having phosphatidylserine and tetraspanin to the amount (A) of the extracellular vesicles having tetraspanin as an indicator.

In the present specification, the cognitive symptom means a condition in which the cognitive function of the brain is reduced due to an acquired organic disorder. Specifically, it is determined that there is a cognitive symptom in a case where the score is 27 points or less out of 30 points, for example, in the Mini-Mental State Examination (MMSE), and it is therefore considered that, for example, Alzheimer's disease or mild cognitive impairment may have occurred. In the present specification, Parkinson's disease with cognitive symptoms is a condition in which a subject has or may develop Parkinson's disease and has or may develop cognitive symptoms, and is, for example, a case of having Parkinson's disease or having a possibility of developing Parkinson's disease and having an MMSE score of 27 points or less.

In the present specification, Parkinson's disease without cognitive symptoms is a condition in which a subject has or may develop Parkinson's disease and has or may develop no cognitive symptoms, and is, for example, a case of having Parkinson's disease or having a possibility of developing Parkinson's disease and having an MMSE score of greater than 27 points.

The determination of Parkinson's disease in the PD diagnosis assisting method (II) is made, for example, by comparing the ratio ((A)/(B)) of the amount (B) of extracellular vesicles having phosphatidylserine and tetraspanin to the amount (A) of extracellular vesicles having tetraspanin, which is obtained by measuring the amount of extracellular vesicles having tetraspanin and measuring the amount of extracellular vesicles having phosphatidylserine and tetraspanin, to a predetermined reference value (cutoff value).

Specifically, in a case where the ratio ((A)/(B)) of the amount (B) of the extracellular vesicles having phosphatidylserine and tetraspanin to the amount (A) of the extracellular vesicles having tetraspanin is equal to or less than a predetermined reference value, it can be determined that a subject is likely to have Parkinson's disease; or a subject may have Parkinson's disease; or a subject is at high risk of developing Parkinson's disease; or a subject is at risk of developing Parkinson's disease.

In addition, in a case where the ratio ((A)/(B)) of the amount (B) of the extracellular vesicles having phosphatidylserine and tetraspanin to the amount (A) of the extracellular vesicles having tetraspanin is larger than a predetermined reference value, it can be determined that a subject is unlikely to have Parkinson's disease; or a subject may not have Parkinson's disease; or a subject is at low risk of developing Parkinson's disease; or a subject is not at risk of developing Parkinson's disease.

The predetermined reference value (cutoff value) in the PD diagnosis assisting method (II) is set in advance to distinguish between a patient with Parkinson's disease and a healthy subject, and is a ratio ((A)/(B)) of an amount (B) of extracellular vesicles having phosphatidylserine and tetraspanin to an amount (A) of extracellular vesicles having tetraspanin in a biological specimen.

The method for determining the predetermined reference value in the PD diagnosis assisting method (II) is not particularly limited. For example, the predetermined reference value can be determined in such a manner that an amount (B) of extracellular vesicles having phosphatidylserine and tetraspanin to an amount (A) of extracellular vesicles having tetraspanin contained in a biological specimen obtained from a patient suffering from Parkinson's disease and a healthy subject is measured to calculate a ratio ((A)/(B)) of the amount (B) of extracellular vesicles having phosphatidylserine and tetraspanin to the amount (A) of extracellular vesicles having tetraspanin, and a statistical analysis such as receiver operating characteristic analysis (ROC analysis) is carried out using the obtained ratio ((A)/(B)) of the amount (B) of extracellular vesicles having phosphatidylserine and tetraspanin to the amount (A) of extracellular vesicles having tetraspanin. In setting the predetermined reference value, it is preferable to consider sensitivity, specificity, positive predictive value, negative predictive value, and the like. For example, the predetermined reference value can be set such that the sensitivity is 60% or more, preferably 70% or more, more preferably 80% or more, and still more preferably 90% or more. For example, the predetermined reference value can be set such that the specificity is 60% or more, preferably 70% or more, more preferably 80% or more, and still more preferably 90% or more.

Using the ratio ((A)/(B)) of the amount (B) of extracellular vesicles having phosphatidylserine and tetraspanin to the amount (A) of extracellular vesicles having tetraspanin as an indicator, the determination of Parkinson's disease in the PD diagnosis assisting method (II) may determine (distinguish) whether the subject is a healthy subject, has Parkinson's disease with cognitive symptoms, or has Parkinson's disease without cognitive symptoms by a plurality of predetermined reference values {cutoff values, for example, a cutoff value for distinguishing between a healthy subject and Parkinson's disease with cognitive symptoms (hereinafter, often referred to simply as "healthy/Parkinson's disease with cognitive symptoms reference value") and a cutoff value for distinguishing between Parkinson's disease with cognitive symptoms and Parkinson's disease without cognitive symptoms (hereinafter, often referred to simply as "Parkinson's disease with cognitive symptoms/Parkinson's disease without cognitive symptoms reference value"); or may determine (distinguish) only whether the subject has Parkinson's disease with cognitive symptoms or Parkinson's disease without cognitive symptoms by the "Parkinson's disease with cognitive symptoms/Parkinson's disease without cognitive symptoms reference value".

In the PD diagnosis assisting method (II), the subject in a case of determining only whether the subject has Parkinson's disease with cognitive symptoms or Parkinson's disease without cognitive symptoms is preferably a human diagnosed with Parkinson's disease based on the diagnostic criteria, or a human diagnosed at risk of developing Parkinson's disease based on diagnostic criteria. The diagnostic criteria are the same as those described above.

The determination of whether the subject has Parkinson's disease with cognitive symptoms or Parkinson's disease without cognitive symptoms in the PD diagnosis assisting method (II) is made, for example, by comparing the ratio ((A)/(B)) of the amount (B) of extracellular vesicles having phosphatidylserine and tetraspanin to the amount (A) of extracellular vesicles having tetraspanin, which is obtained by measuring the amount of extracellular vesicles having tetraspanin and measuring the amount of extracellular vesicles having phosphatidylserine and tetraspanin, with a predetermined "Parkinson's disease with cognitive symptoms/Parkinson's disease without cognitive symptoms reference value"

Specifically, in a case where the ratio ((A)/(B)) of the amount (B) of the extracellular vesicles having phosphatidylserine and tetraspanin to the amount (A) of the extracellular vesicles having tetraspanin is equal to or less than the "Parkinson's disease with cognitive symptoms/Parkinson's disease without cognitive symptoms reference value", it can be determined that a subject is likely to have Parkinson's disease without cognitive symptoms; or a subject is at high risk of developing Parkinson's disease without cognitive symptoms; or a subject is at risk of developing Parkinson's disease without cognitive symptoms.

In addition, in a case where the ratio ((A)/(B)) of the amount (B) of the extracellular vesicles having phosphatidylserine and tetraspanin to the amount (A) of the extracellular vesicles having tetraspanin is larger than the "Parkinson's disease with cognitive symptoms/Parkinson's disease without cognitive symptoms reference value", it can be determined that a subject is likely to have Parkinson's disease with cognitive symptoms; or a subject is at high risk of developing Parkinson's disease with cognitive symptoms; or a subject is at risk of developing Parkinson's disease with cognitive symptoms.

The method for determining the "Parkinson's disease with cognitive symptoms/Parkinson's disease without cognitive symptoms reference value" is not particularly limited. For example, the predetermined reference value can be determined in such a manner that an amount (B) of extracellular vesicles having phosphatidylserine and tetraspanin to an amount (A) of extracellular vesicles having tetraspanin contained in a biological specimen obtained from a patient suffering from Parkinson's disease with cognitive symptoms and a patient suffering from Parkinson's disease without cognitive symptoms is measured to calculate a ratio ((A)/(B)) of the amount (B) of extracellular vesicles having phosphatidylserine and tetraspanin to the amount (A) of extracellular vesicles having tetraspanin, and a statistical analysis such as receiver operating characteristic analysis (ROC analysis) is carried out using the obtained ratio ((A)/(B)) of the amount (B) of extracellular vesicles having phosphatidylserine and tetraspanin to the amount (A) of extracellular vesicles having tetraspanin. In setting the predetermined reference value, it is preferable to consider sensitivity, specificity, positive predictive value, negative predictive value, and the like. For example, the predetermined reference value can be set such that the sensitivity is 60% or more, preferably 70% or more, more preferably 80% or more, and still more preferably 90% or more. For example, the predetermined reference value can be set such that the specificity is 60% or more, preferably 70% or more, more preferably 80% or more, and still more preferably 90% or more.

The determination of whether the subject in the PD diagnosis assisting method (II) is a healthy subject, has Parkinson's disease with cognitive symptoms, or has Parkinson's disease without cognitive symptoms can be made in such a manner that, for example, in a case where the ratio ((A)/(B)) of the amount (B) of the extracellular vesicles having phosphatidylserine and tetraspanin to the amount (A) of the extracellular vesicles having tetraspanin is equal to or less than the "Parkinson's disease with cognitive symptoms/Parkinson's disease without cognitive symptoms reference value", a subject is likely to have Parkinson's disease without cognitive symptoms; or a subject is at high risk of developing Parkinson's disease without cognitive symptoms; or a subject is at risk of developing Parkinson's disease without cognitive symptoms; in a case where the ratio ((A)/(B)) is greater than the "Parkinson's disease with cognitive symptoms/Parkinson's disease without cognitive symptoms reference value" and is equal to or less than the "healthy/Parkinson's disease with cognitive symptoms reference value", a subject is likely to have Parkinson's disease with cognitive symptoms; or a subject is at high risk of developing Parkinson's disease with cognitive symptoms; or a subject is at risk of developing Parkinson's disease with cognitive symptoms; and in a case where the ratio ((A)/(B)) is greater than the "healthy/Parkinson's disease with cognitive symptoms reference value", a subject is unlikely to have Parkinson's disease; or a subject may not have Parkinson's disease; or a subject is at low risk of developing Parkinson's disease; or a subject is not at risk of developing Parkinson's disease.

The method for determining the "healthy/Parkinson's disease with cognitive symptoms reference value" is not particularly limited. For example, the predetermined reference value can be determined in such a manner that an amount (B) of extracellular vesicles having phosphatidylserine and tetraspanin to an amount (A) of extracellular vesicles having tetraspanin contained in a biological specimen obtained from a healthy subject and a patient with Parkinson's disease with cognitive symptoms is measured to calculate a ratio ((A)/(B)) of the amount (B) of extracellular vesicles having phosphatidylserine and tetraspanin to the amount (A) of extracellular vesicles having tetraspanin, and a statistical analysis such as receiver operating characteristic analysis (ROC analysis) is carried out using the obtained ratio ((A)/(B)) of the amount (B) of extracellular vesicles having phosphatidylserine and tetraspanin to the amount (A) of extracellular vesicles having tetraspanin. In setting the predetermined reference value, it is preferable to consider sensitivity, specificity, positive predictive value, negative predictive value, and the like. For example, the predetermined reference value can be set such that the sensitivity is 60% or more, preferably 70% or more, more preferably 80% or more, and still more preferably 90% or more. For example, the predetermined reference value can be set such that the specificity is 60% or more, preferably 70% or more, more preferably 80% or more, and still more preferably 90% or more.

### Use of a reagent kit (II) for assisting diagnosis of Parkinson's disease

The reagent kit for assisting the diagnosis of Parkinson's disease in the second invention (hereinafter, often referred to simply as "reagent kit (II) for assisting the diagnosis of PD") contains a substance having an affinity for tetraspanin and a substance having an affinity for phosphatidylserine.

The substance having an affinity for tetraspanin and the substance having an affinity for phosphatidylserine in the reagent kit (II) for assisting the diagnosis of PD are the same as those described above in the PD diagnosis assisting method (II), and preferred ones thereof are also the same. The substance having an affinity for tetraspanin and the substance having an affinity for phosphatidylserine each may be in a solution state, a frozen state, a dried state, or a freeze-dried state. In addition, the substance having an affinity for tetraspanin and the substance having an affinity for phosphatidylserine may be contained in the kit as one reagent or may be contained in the kit as separate reagents.

The combination of the substance having an affinity for tetraspanin and the substance having an affinity for phosphatidylserine in the reagent kit (II) for assisting the diagnosis of PD includes the same combinations as the combinations described above in the PD diagnosis assisting method (II), and preferred combinations thereof are also the same.

The substance having an affinity for tetraspanin or/and the substance having an affinity for phosphatidylserine in the reagent kit (II) for assisting the diagnosis of PD may be immobilized on a solid phase and may be labeled with a labeling substance. The solid phase, the method for immobilizing the substance on the solid phase, the labeling substance, and the labeling method are the same as those described above in the PD diagnosis assisting method, and preferred ones thereof are also the same.

The reagent kit (II) for assisting the diagnosis of PD may further contain a secondary affinity substance (for example, a secondary antibody) that specifically binds to the substance having an affinity for tetraspanin or/and the substance having an affinity for phosphatidylserine. The secondary affinity substance in the reagent kit (II) for assisting the diagnosis of PD is the same as that described above in the PD diagnosis assisting method, and preferred ones thereof are also the same. In addition, the secondary affinity substance in the reagent kit (II) for assisting the diagnosis of PD may be labeled with a labeling substance. The labeling substance and the labeling method are the same as those described above in the PD diagnosis assisting method and preferred ones thereof are also the same.

The substance having an affinity for tetraspanin or/and the substance having an affinity for phosphatidylserine in the reagent kit (II) for assisting the diagnosis of PD may be a substance to which one of avidin and biotin is bound. The avidin and the biotin are the same as those described above in the PD diagnosis assisting method, and preferred ones thereof are also the same.

The reagent kit (II) for assisting the diagnosis of PD may contain a labeling substance to which one of avidin and biotin is bound. The labeling substance and labeling method are the same as those described above in the PD diagnosis assisting method, and preferred ones thereof are also the same.

The concentration (amount) of the substance having an affinity for tetraspanin and the substance having an affinity for phosphatidylserine in the reagent kit (II) for assisting the diagnosis of PD may be appropriately set within the range commonly used in this field depending on the measuring method. For example, the substance having an affinity for tetraspanin is contained at a concentration at the time of use of usually 10 to 20,000 ng/mL and preferably 100 to 10,000 ng/mL, in a case of being immobilized on a solid phase, and at a concentration of usually 10 to 5,000 ng/mL and preferably 100 to 500 ng/mL in a case of being used for detection. In addition, for example, the substance having an affinity for phosphatidylserine is contained at a concentration at the time of use of usually 10 to 20,000 ng/mL and preferably 100 to 10,000 ng/mL, in a case of being immobilized on a solid phase, and at a concentration of usually 10 to 5,000 ng/mL and preferably 100 to 500 ng/mL in a case of being used for detection.

In addition, the substance having an affinity for tetraspanin and the substance having an affinity for phosphatidylserine in the reagent kit (II) for assisting the diagnosis of PD may coexist with a reagent commonly used in this field. The reagent is the same as that of the reagent kit for assisting the diagnosis of PD.

Further, the reagent kit (II) for assisting the diagnosis of PD may contain, in addition to the substance having an affinity for tetraspanin and the substance having an affinity for phosphatidylserine, a reagent needed to measure an amount of extracellular vesicles having tetraspanin and phosphatidylserine or/and amount of extracellular vesicles having phosphatidylserine using these affinity substances. Such a reagent is the same as that of the reagent kit for assisting the diagnosis of PD.

The reagent kit (II) for assisting the diagnosis of PD may contain a reference standard used for creating a calibration curve for extracellular vesicles having phosphatidylserine and tetraspanin. The reference standard is the same as that of the reagent kit for assisting the diagnosis of PD.

In addition, the reagent kit (II) for assisting the diagnosis of PD may contain a reference standard used for creating a calibration curve for extracellular vesicles having tetraspanin. Examples of the reference standard include an extracellular vesicle having tetraspanin. The reference standard may be in a solution state, a frozen state, a dried state, or a freeze-dried state. In addition, reagents commonly used in this field, for example, a buffer, a reaction promoter, a sugar, a protein, a salt, a stabilizer such as a surfactant, and a preservative, may coexist with the reference standard. The concentration and pH of these reagents may be appropriately selected from the ranges commonly used in this field.

The reagent kit (II) for assisting the diagnosis of PD may contain a package insert or an instruction manual. Examples of the package insert or the instruction manual include a package insert or instruction manual stating that a subject is determined to have Parkinson's disease by measuring an amount of extracellular vesicles having tetraspanin and an amount of extracellular vesicles having phosphatidylserine and tetraspanin in a biological specimen derived from the subject or/and using a ratio of the amount of extracellular vesicles having phosphatidylserine and tetraspanin to the amount of extracellular vesicles having tetraspanin as an indicator (for example, PD diagnosis assisting method (II)). These package insert and instruction manual may be described separately in a plurality of cases, or may be described collectively in one.

Specific examples of the reagent kit (II) for assisting the diagnosis of PD include a kit containing the following (A) and (B).
(A) one of a substance having an affinity for tetraspanin and a substance having an affinity for phosphatidylserine immobilized on a solid phase and the other one of the substance having an affinity for tetraspanin and the substance having an affinity for phosphatidylserine bound to a labeling substance or the other one affinity substance and a component for indirectly binding the other one affinity substance to a labeling substance.
(B) a substance having an affinity for tetraspanin immobilized on a solid phase and a substance having an affinity for tetraspanin bound to a labeling substance or the affinity substance and a component for indirectly binding the affinity substance to a labeling substance.

The reagent kit (II) for assisting the diagnosis of PD is preferably, for example, a kit containing the following (C) and (D).

(C) one of a substance having an affinity for tetraspanin and a substance having an affinity for phosphatidylserine immobilized on a solid phase and one selected from the following (1) to (3),
(1) the other one of the substance having an affinity for tetraspanin and the substance having an affinity for phosphatidylserine bound to a labeling substance, (2) the other one of the substance having an affinity for tetraspanin and the substance having an affinity for phosphatidylserine, and a secondary affinity substance that specifically binds to the affinity substance bound to a labeling substance, and (3) the other one of the substance having an affinity for tetraspanin and the substance having an affinity for phosphatidylserine to which one of avidin and biotin is bound, and a labeling substance to which the other one of avidin and biotin is bound.

(D) a substance having an affinity for tetraspanin immobilized on a solid phase and one selected from the following (4) to (6),
one selected from (4) a substance having an affinity for tetraspanin bound to a labeling substance, (5) a substance having an affinity for tetraspanin, and a secondary affinity substance, which specifically binds to a substance having an affinity for tetraspanin, bound to a labeling substance, and (6) a substance having an affinity for tetraspanin bound to one of avidin and biotin, and the other one of avidin and biotin bound to a labeling substance.

Preferred examples of the reagent kit (II) for assisting the diagnosis of PD include the following.

For example, there is a kit containing a solid phase plate on which an antibody or Tim protein (more preferably Tim4 or Tim1 and particularly preferably the Tim4) that specifically binds to phosphatidylserine is immobilized, a solid phase plate on which a tetraspanin antibody (more preferably an anti-CD9 antibody or an anti-CD63 antibody and particularly preferably the anti-CD9 antibody) is immobilized, and any one selected from the following (1) to (3).
(1) a solution containing a tetraspanin antibody (more preferably an anti-CD9 antibody or an anti-CD63 antibody and particularly preferably the anti-CD9 antibody) bound to a labeling substance (usually 10 to 5,000 ng/mL and preferably 100 to 500 ng/mL), (2) a solution containing a tetraspanin antibody (more preferably an anti-CD9 antibody or an anti-CD63 antibody and particularly preferably the anti-CD9 antibody) (usually 10 to 5,000 ng/mL and preferably 100 to 500 ng/mL), and a solution containing a secondary affinity substance that specifically binds to the tetraspanin antibody bound to a labeling substance (usually 10 to 5,000 ng/mL and preferably 100 to 500 ng/mL), and (3) a solution containing an antibody that specifically binds to tetraspanin (preferably an anti-CD9 antibody or an anti-CD63 antibody and more preferably the anti-CD9 antibody) to which one of avidin and biotin is bound (usually 10 to 5,000 ng/mL and preferably 100 to 500 ng/mL), and a solution containing a labeling substance to which the other one of avidin and biotin is bound (usually 10 to 5,000 ng/mL and preferably 100 to 500 ng/mL).

### Device (II) for assisting diagnosis of Parkinson's disease (falling outside the scope of the claims)

The device for assisting the diagnosis of Parkinson's disease in the second invention (hereinafter, often referred to simply as "device (II) for assisting the diagnosis of PD") has a measurement unit that measures an amount of extracellular vesicles having phosphatidylserine and tetraspanin and an amount of extracellular vesicles having tetraspanin, in a biological specimen derived from a subject; a calculation unit that calculates a ratio of the amount of extracellular vesicles having phosphatidylserine and tetraspanin to the amount of extracellular vesicles having tetraspanin; and a determination unit that determines that the subject has Parkinson's disease using the ratio of the amount of extracellular vesicles having phosphatidylserine and tetraspanin in the biological specimen to the amount of extracellular vesicles having tetraspanin in the biological specimen as an indicator.

The measurement unit, the calculation unit, the determination unit, and the like constituting the device (II) for assisting the diagnosis of PD may be arranged in the same device or may be separate bodies.

The measurement unit in the device (II) for assisting the diagnosis of PD is a site for measuring the amount of extracellular vesicles having phosphatidylserine and tetraspanin and the amount of extracellular vesicles having tetraspanin in the biological specimen introduced into the device. The size and configuration of the measurement unit are not particularly limited.

Examples of the measurement unit include an imaging apparatus for imaging using a microplate reader or CCD used for ELISA, an imaging apparatus used for a method using Western blotting or a microarray (microchip), a mass spectrometer used for mass spectrometry, an intermolecular interaction analyzer, a flow cytometer used for flow cytometry, and an ultraviolet/visible light detector or fluorescence detector used for HPLC or capillary electrophoresis.

The subject, the biological specimen, and the extracellular vesicle in the device (II) for assisting the diagnosis of PD are the same as those described above, and preferred ones thereof are also the same.

The extracellular vesicle having phosphatidylserine and tetraspanin, the amount, and the target for measuring the amount of extracellular vesicles having phosphatidylserine and tetraspanin in the device (II) for assisting the diagnosis of PD are the same as those described above in the PD diagnosis assisting method, and preferred ones thereof are also the same.

The measurement of the amount of extracellular vesicles having phosphatidylserine and tetraspanin and the substances used for the measurement in the device (II) for assisting the diagnosis of PD are the same as those described above in the PD diagnosis assisting method, and preferred ones and specific examples thereof are also the same.

In addition, the extracellular vesicle having tetraspanin and the target for measuring the amount of extracellular vesicles having tetraspanin in the device (II) for assisting the diagnosis of PD are the same as those described above in the PD diagnosis assisting method (II), and preferred ones thereof are also the same.

The measurement of the amount of extracellular vesicles having tetraspanin and the substances used for the measurement in the device (II) for assisting the diagnosis of PD are the same as those described above in the PD diagnosis assisting method (II), and preferred ones and specific examples thereof are also the same.

The calculation unit in the device (II) for assisting the diagnosis of PD is a site for calculating the ratio of the amount of extracellular vesicles having phosphatidylserine and tetraspanin to the amount of extracellular vesicles having tetraspanin, based on the amount of extracellular vesicles having tetraspanin and the amount of extracellular vesicles having phosphatidylserine and tetraspanin obtained by the measurement unit. The size and configuration of the measurement unit are not particularly limited.

The calculation unit in the device (II) for assisting the diagnosis of PD may convert an actually measured value (for example, an absorbance, an amount of change in absorbance, transmitted light, an amount of change in transmitted light, a fluorescence intensity, an amount of change in fluorescence intensity, an amount of luminescence, an amount of change in amount of luminescence, a turbidity, rate of change in turbidity, scattered light, a rate of change in scattered light, a reflectivity, an amount of change in reflectivity, a refractive index, or an amount of change in refractive index) having a correlation with the mass or concentration of extracellular vesicles having phosphatidylserine and tetraspanin and the extracellular vesicles having tetraspanin in the biological specimen obtained by the measurement unit in the device for assisting the diagnosis of PD into mass, concentration, or the like, and then calculate the ratio of the amount of extracellular vesicles having phosphatidylserine and tetraspanin to the amount of extracellular vesicles having tetraspanin.

It should be noted that the actually measured value, the mass, concentration, or the like converted by the calculation unit, and the ratio of the amount of extracellular vesicles having phosphatidylserine and tetraspanin to the amount of extracellular vesicles having tetraspanin may be stored in a storage device or the like provided in a device such as a memory device or a hard disk.

The determination unit in the device (II) for assisting the diagnosis of PD is a site for determining Parkinson's disease using the ratio of the amount of extracellular vesicles having phosphatidylserine and tetraspanin to the amount of extracellular vesicles having tetraspanin calculated by the calculation unit as an indicator.

In addition, the determination unit in the device (II) for assisting the diagnosis of PD may be a site for distinguishing and determining whether the subject has Parkinson's disease with cognitive symptoms or Parkinson's disease without cognitive symptoms.

The determination of Parkinson's disease and the predetermined reference value used for the determination in the device (II) for assisting the diagnosis of PD, and the method for determining the predetermined reference value are the same as those described above in the PD diagnosis assisting method (II) and preferred ones and specific examples thereof are also the same.

The predetermined reference value in the device (II) for assisting the diagnosis of PD may be stored in advance in the device (II) for assisting the diagnosis of PD, or may be input from an input site of the device (II) for assisting the diagnosis of PD at the time of determination.

The device (II) for assisting the diagnosis of PD may contain an output unit. The output unit carries out processing such as displaying or outputting the results of the determination to a display device such as a display or a printing device such as a printer.

According to the present invention, data (determination result) for assisting the diagnosis of a subject regarding Parkinson's disease by a physician can be obtained.

In a case where the subject is determined to have Parkinson's disease by the present invention, the physician may further carry out, for example, a medical interview, a test on a biomarker or the like of Parkinson's disease recommended in the Parkinson's disease clinical practice guideline such as MIBG myocardial scintigraphy or dopamine transporter scintigraphy, or a test using candidate substances for the biomarker of Parkinson's disease, and can make a diagnosis of Parkinson's disease of the subject in consideration of the results thereof and the like.

In addition, in a case where the subject is determined to have Parkinson's disease by the present invention, a drug for Parkinson's disease (a drug or a therapeutic agent for delaying the progression of Parkinson's disease) such as levodopa (L-dopa) may be administered, or surgery may be carried out.

In a case where the subject is determined to have Parkinson's disease with cognitive symptoms, it is preferable not to administer a drug that may adversely affect cognitive symptoms such as an anticholinergic drug, and it is preferable to administer a drug for dementia or mild cognitive impairment (a drug or therapeutic agent that delays the progression of dementia or mild cognitive impairment) such as a cholinesterase inhibitor, or carry out surgery.

In a case where the subject is determined to have Parkinson's disease without cognitive symptoms, even a drug that may adversely affect cognitive symptoms, such as an anticholinergic drug, can be administered.

Therefore, determining (distinguishing) whether a subject has Parkinson's disease without cognitive symptoms or Parkinson's disease with cognitive symptoms is useful for a physician to determine a therapeutic strategy or make a diagnosis.

### Industrial Applicability

The method for assisting the diagnosis of Parkinson's disease, the biomarker, the reagent kit, and the device according to the present invention can assist in the diagnosis of Parkinson's disease and are therefore useful in the field of clinical examination. According to the present invention, it is possible to assist in the diagnosis of Parkinson's disease with high accuracy.

### Examples

Hereinafter, the present invention will be described in more detail based on Examples and Comparative Examples, but the present invention is not limited to these examples. In Examples and Comparative Examples, the "patient with Parkinson's disease (MMSE score of greater than 27)" is a test specimen of a patient with Parkinson's disease without cognitive symptoms, and the "patient with Parkinson's disease (MMSE score of 27 or less)" is a test specimen of a patient with Parkinson's disease with cognitive symptoms.

### Example 1. Evaluation of Parkinson's disease test specimen (MMSE score of greater than 27) using amount of exosomes having PS and CD9 as indicator

Exosomes were measured by sandwich ELISA of Tim protein-anti-CD9 antibody, and AUC and p-value were calculated.

### (1) Preparation of calibrator

Using MagCapture (registered trademark) Exosome Isolation Kit PS (manufactured by FUJIFILM Wako Pure Chemical Corporation, hereinafter referred to as "A kit"), exosomes were isolated from COLO201 cell culture supernatant according to the instruction manual attached to the kit, and the exosomes were eluted using an eluent attached to the kit. Using a protein assay BCA kit (manufactured by FUJIFILM Wako Pure Chemical Corporation), a protein concentration in the obtained exosome solution was measured by a bicinchoninic acid method (BCA method).

Next, using Reaction Buffer attached to PS Capture Exosome ELISA Kit, Streptavidin HRP (manufactured by FUJIFILM Wako Pure Chemical Corporation, hereinafter referred to as "B kit"), the obtained exosome solution was diluted to 0.156, 0.313, 0.625, 1.25, 2.50, 5.00, 10.0, and 20.0 ng/mL, based on the protein concentration measured by the BCA method, whereby an exosome dilution series consisting of 8-point concentrations (hereinafter, referred to as "calibrator") derived from the COLO201 cell culture supernatant was obtained.

### (2) Pretreatment of test specimen for measurement

8 test specimens of patients with Parkinson's disease (MMSE score of greater than 27) and EDTA plasma of 37 test specimens of healthy subjects purchased from PrecisionMed, LLC. were each centrifuged at 10,000 g for 20 minutes, and the supernatant was collected. Each of the obtained supernatants was diluted 100-fold with Reaction Buffer attached to the A kit to obtain a "test specimen diluted solution for measurement".

### (3) Measurement by ELISA

The test specimen diluted solution for measurement prepared in (2) was measured by sandwich ELISA of Tim4 protein-anti-CD9 antibody in which the Tim4 protein was immobilized on a solid phase and the anti-CD9 antibody was used as an antibody for detection. Specifically, an anti-CD9 mouse monoclonal antibody (1K, manufactured by FUJIFILM Wako Pure Chemical Corporation) was reduced with 1.6 mM DTT and then reacted with Biotin-PEAC5-maleimide (manufactured by Dojindo Laboratories) at 37°C for 1.5 hours to prepare a biotin-labeled anti-CD9 mouse monoclonal antibody. The reagents attached to the B kit were used, except for the antibody for detection.

First, Washing Buffer (10×) attached to the B kit was diluted 10-fold with purified water (distilled water), and then Exosome Binding Enhancer (100×) attached to the B kit was added in an amount of 1/100 to the obtained diluted solution. The obtained solution is referred to as "washing solution (1x)". Next, each well of a "96-well plate in which the Tim4 protein was immobilized on a solid phase" attached to the B kit was washed 3 times with 300 to 350 µL of the washing solution (1×).

Next, the test specimen diluted solution for measurement (8 test specimens, n = 2, 16 wells) of patients with Parkinson's disease (MMSE score of greater than 27) and the test specimen diluted solution for measurement of healthy subject test specimens (37 test specimens, n = 2, 74 wells) prepared in (2), the calibrator prepared in (2) (8 points, n = 2, 16 wells), and Reaction Buffer (n = 2, 2 wells) attached to the B kit as a blank were dispensed at an amount of 100 µL/well into each well of the plate. Then, a plate seal was attached to the plate, followed by reaction at room temperature for 2 hours while stirring at about 500 rpm using a microplate shaker. After the reaction was completed, the reaction solution was discarded and each well was washed 3 times with 300 to 350 µL of the washing solution (1×). Then, using Reaction Buffer attached to the B kit, the biotin-labeled anti-CD9 mouse monoclonal antibodies were diluted to a final concentration of 250 ng/mL to obtain a biotin-labeled antibody reaction solution. The obtained biotin-labeled antibody reaction solution was dispensed at an amount of 100 µL/well into each well of the plate, and a plate seal was attached to the plate, followed by reaction at room temperature for 1 hour while stirring at about 500 rpm using a microplate shaker. After the reaction was completed, the reaction solution was discarded and each well was washed 3 times with 300 to 350 µL of the washing solution (1×).

HRP-conjugated streptavidin (100×) in an amount of 1/100 was added to Reaction Buffer attached to the B kit, which was then thoroughly mixed to prepare an HRP-labeled streptavidin reaction solution (1×). The obtained HRP-labeled streptavidin reaction solution (1×) was dispensed at an amount of 100 µL/well into each well of the plate, and a plate seal was attached to the plate, followed by reaction at room temperature for 2 hours while stirring at about 500 rpm using a microplate shaker. After the reaction was completed, the reaction solution was discarded and each well was washed 5 times with 300 to 350 µL of the washing solution (1×).

Next, the 3,3',5,5'-tetramethylbenzidine (TMB) solution attached to the B kit, which was returned to room temperature, was dispensed at an amount of 100 µL/well into each well of the plate, followed by stirring for about 1 minute using a microplate shaker. After that, a plate seal was attached to the plate which was then allowed to stand at room temperature (20°C to 25°C) for 30 minutes. Then, the stop solution attached to the B kit, which was returned to room temperature, was dispensed at an amount of 100 µL/well into each well of the plate, followed by stirring for about 5 seconds using a microplate shaker, and the absorbance at 450 nm and the absorbance at a sub-wavelength of 620 nm were immediately measured using a 96-well microplate reader (Safire2, available from Tecan Group Ltd.). The value obtained by subtracting the absorbance value at a sub-wavelength of 620 nm from the absorbance value at 450 nm was defined as an "absorbance value", and the value obtained by subtracting the blank absorbance value from the absorbance value of the test specimen diluted solution for measurement was defined as a "corrected test specimen absorbance value". Then, a standard curve was created from the value obtained by subtracting the blank absorbance value from the absorbance value of the exosome dilution series (calibrator) derived from COLO201 cell culture supernatant and the protein concentration of the calibrator. The corrected test specimen absorbance value was converted into a protein concentration using the standard curve, and the value obtained by multiplying the obtained corresponding value by the test specimen dilution rate was defined as a "test specimen measurement value" [ng/mL].

### (4) Calculation of AUC

Based on the test specimen measurement value obtained in (3), a significant difference test between a patient with Parkinson's disease and a healthy subject was carried out by the Wilcoxon/Kruskal-Wallis test (rank sum) using JMP (registered trademark) 11 (available from SAS Institute Inc., Cary, NC, USA), and a p-value was calculated. In addition, based on the test specimen measurement value obtained in (3), a logistic regression analysis was carried out using JMP (registered trademark) 11 (available from SAS Institute Inc., Cary, NC, USA), and an area under the curve (AUC) value was calculated from the obtained receiver operating characteristic curve (ROC curve).

The obtained results are shown in Table 1 which will be given later. In addition, Fig. 1 shows a box plot graph created based on the test specimen measurement value. In the figure, the vertical axis shows the test specimen measurement value, and Control on the horizontal axis shows the results of a healthy subject, and PD on the horizontal axis shows the results of a patient with Parkinson's disease (MMSE score of greater than 27).

### Comparative Example 1. Evaluation of Parkinson's disease test specimen (MMSE score of greater than 27) using amount of exosomes having CD9 as indicator

Exosomes were measured by sandwich ELISA of anti-CD9 antibody-anti-CD9 antibody in the same manner as in Example 1, except that an anti-CD9 antibody was immobilized on a solid phase instead of the Tim4 protein. Then, a significant difference test between a patient with Parkinson's disease (MMSE score of greater than 27) and a healthy subject was carried out, and the AUC and p-value were calculated.

A plate in which the anti-CD9 antibody was immobilized on a solid phase was prepared by the following method. Anti-CD9 mouse monoclonal antibodies (1K) (manufactured by FUJIFILM Wako Pure Chemical Corporation) were diluted with 50 mM MOPS (pH 7.5) to a concentration of 10 µg/mL, and were added at an amount of 100 µL/well to wells of a 96-well microplate (available from Nunc Inc.) which was then incubated overnight in a refrigerator. The wells were washed three times with TBST (Tris Buffered Saline, pH 7.4), 300 µL of TBS (Tris Buffered Saline, pH 7.4) containing 10 mg/mL of Block Ace was added thereto, followed by incubation overnight in a refrigerator. The thus-treated plate was used as an antibody-immobilized plate.

As the anti-CD9 antibody of the detection antibody, a biotin-labeled antibody was used in the same manner as in Example 1.

The obtained results are shown in Table 1 which will be given later. In addition, Fig. 1 shows a box plot graph created based on the test specimen measurement value. In the figure, the vertical axis shows the test specimen measurement value, and Control on the horizontal axis shows the results of a healthy subject, and PD on the horizontal axis shows the results of a patient with Parkinson's disease (MMSE score of greater than 27).

### Example 2. Evaluation of Parkinson's disease test specimen (MMSE score of greater than 27) using amount of exosomes having PS and CD63 as indicator

Exosomes were measured by sandwich ELISA of Tim protein-anti-CD63 antibody in the same manner as in Example 1, except that an anti-CD63 antibody was used instead of the anti-CD9 antibody as the detection antibody. Then, a significant difference test between a patient with Parkinson's disease (MMSE score of greater than 27) and a healthy subject was carried out, and the AUC and p-value were calculated.

The biotin-labeled anti-CD63 antibody attached to PS Capture Exosome ELISA Kit, Streptavidin HRP (manufactured by FUJIFILM Wako Pure Chemical Corporation, the "B kit" described above) was used as the anti-CD63 antibody of the detection antibody.

The obtained results are shown in Table 1 which will be given later.

### Comparative Example 2. Evaluation of Parkinson's disease test specimen using CD63 as indicator

Exosomes were measured by sandwich ELISA of anti-CD63 antibody-anti-CD63 antibody in the same manner as in Comparative Example 1, except that an anti-CD63 mouse monoclonal antibody (3-13) (manufactured by FUJIFILM Wako Pure Chemical Corporation) was immobilized on a solid phase instead of the anti-CD9 antibody and used as the detection antibody. Then, a significant difference test between a patient with Parkinson's disease (MMSE score of greater than 27) and a healthy subject was carried out, and the AUC and p-value were calculated.

A plate in which the anti-CD63 antibody was immobilized on a solid phase was prepared in the same manner as in Comparative Example 1. The biotin-labeled anti-CD63 antibody attached to PS Capture Exosome ELISA Kit, Streptavidin HRP (manufactured by FUJIFILM Wako Pure Chemical Corporation, the "B kit" described above) was used as the anti-CD63 antibody of the detection antibody.

The obtained results are shown in Table 1 which will be given later.

### Example 3. Evaluation of Parkinson's disease dementia specimen using amount of exosomes having PS and CD81 as indicator

Exosomes were measured by sandwich ELISA of Tim protein-anti-CD81 antibody in the same manner as in Example 1, except that an anti-CD81 mouse monoclonal antibody (17B1) (manufactured by FUJIFILM Wako Pure Chemical Corporation) was used instead of the anti-CD9 antibody as the detection antibody. Then, a significant difference test between a patient with Parkinson's disease (MMSE score of greater than 27) and a healthy subject was carried out, and the AUC and p-value were calculated.

As the anti-CD81 antibody of the detection antibody, a biotin-labeled antibody was used in the same manner as in Example 1.

The obtained results are shown in Table 1 which will be given later.

### Comparative Example 3. Evaluation of Parkinson's disease test specimen (MMSE score of greater than 27) using amount of exosomes having CD81 as indicator

Exosomes were measured by sandwich ELISA of anti-CD81 antibody-anti-CD81 antibody in the same manner as in Example 1, except that an anti-CD81 mouse monoclonal antibody (17B1) (manufactured by FUJIFILM Wako Pure Chemical Corporation) was immobilized on a solid phase instead of the anti-CD9 antibody and used as the detection antibody. Then, a significant difference test between a patient with Parkinson's disease (MMSE score of greater than 27) and a healthy subject was carried out, and the AUC and p-value were calculated.

A plate in which the anti-CD81 antibody was immobilized on a solid phase was prepared in the same manner as in Comparative Example 1.

As the anti-CD81 antibody of the detection antibody, a biotin-labeled antibody was used in the same manner as in Example 1.

The obtained results are shown in Table 1 which will be given later.

Example 4. Evaluation of Parkinson's disease test specimen (MMSE score of greater than 27) using ratio of amount of exosomes having PS and CD9 to amount of exosomes having CD9 as indicator

The "test specimen measurement value" (value (A)) obtained in Example 1 (sandwich ELISA of Tim protein-anti-CD9 antibody) was divided by the "test specimen measurement value" (value (B)) obtained in Comparative Example 1 (sandwich ELISA of anti-CD9 antibody-anti-CD9 antibody) to obtain (A)/(B) (hereinafter, referred to as "corrected test specimen measurement value").

Based on the obtained corrected test specimen measurement value, a significant difference test between a patient with Parkinson's disease (MMSE score of greater than 27) and a healthy subject was carried out in the same manner as in Example 1 (4), and the AUC and p-value were calculated.

The obtained results are shown in Table 1 which will be given later. In addition, Fig. 2 shows a box plot graph created based on the corrected test specimen measurement value. In the figure, the vertical axis shows the corrected test specimen measurement value ((A)/(B)), and Control on the horizontal axis shows the results of a healthy subject, and PD on the horizontal axis shows the results of a patient with Parkinson's disease (MMSE score of greater than 27).

Example 5. Evaluation of Parkinson's disease test specimen (MMSE score of greater than 27) using ratio of amount of exosomes having PS and CD63 to amount of exosomes having CD63 as indicator

The "test specimen measurement value" (value (A)) obtained in Example 2 (sandwich ELISA of Tim protein-anti-CD63 antibody) was divided by the "test specimen measurement value" (value (B)) obtained in Comparative Example 2 (sandwich ELISA of anti-CD63 antibody-anti-CD63 antibody) to obtain (A)/(B) (corrected test specimen measurement value).

Based on the obtained corrected test specimen measurement value, a significant difference test between a patient with Parkinson's disease (MMSE score of greater than 27) and a healthy subject was carried out in the same manner as in Example 1 (4), and the AUC and p-value were calculated.

The obtained results are shown in Table 1 which will be given later.

**Table 1**

| | Specimen | Solid phase | Detection | Test specimen measurement value used for analysis | Healthy subject-PD (MMSE score of greater than 27) | |
|---|---|---|---|---|---|---|
| | | | | | AUC | p-value |
| Example 1 | | Tim4 | αCD9 | Value (A) | 0.929 | 0.0002 |
| Comparative Example 1 | | αCD9 | αCD9 | Value (B) | 0.855 | 0.002 |
| Example 4 | | | | Value (A)/Value (B) | 0.973 | <0.0001 |
| Example 2 | Plasma | Tim4 | αCD63 | Value (A) | 0.899 | 0.001 |
| Comparative Example 2 | | αCD63 | αCD63 | Value (B) | 0.848 | 0.002 |
| Example 5 | | | | Value (A) Value (B) | 0.892 | 0.001 |
| Example 3 | | Tim4 | αCD81 | Value (A) | 0.916 | 0.0003 |
| Comparative Example 3 | | αCD81 | αCD81 | Value (B) | 0.642 | 0.218 |

From Table 1, in a case where Parkinson's disease was evaluated using plasma derived from a patient with Parkinson's disease without cognitive symptoms (MMSE score of greater than 27) and a healthy subject as the specimen and using the amount of exosomes having tetraspanin of CD9, CD63, or CD81 as an indicator, the AUCs were 0.855, 0.848, and 0.642, respectively.

On the other hand, in a case where Parkinson's disease was evaluated using plasma derived from a patient with Parkinson's disease without cognitive symptoms (MMSE score of greater than 27) and a healthy subject as the specimen and using the amount of exosomes having phosphatidylserine to which tetraspanin of CD9, CD63, or CD81 and Tim protein bind as an indicator, the AUCs were 0.929, 0.899, and 0.916, respectively, and therefore it was found that Parkinson's disease (particularly Parkinson's disease without cognitive symptoms) can be detected with high accuracy in any of those cases.

In addition, in a case where Parkinson's disease was evaluated using the corrected test specimen measurement value ((A)/(B)) obtained by dividing the test specimen measurement value (value (A)) of the exosome having phosphatidylserine and CD63 by the test specimen measurement value (value (B)) of the exosome having CD63 as an indicator, the AUC was 0.892, and therefore it was found that Parkinson's disease (particularly Parkinson's disease without cognitive symptoms) can be detected with high accuracy. In addition, in a case where Parkinson's disease was evaluated using the corrected test specimen measurement value ((A)/(B)) obtained by dividing the test specimen measurement value (value (A)) of the exosome having phosphatidylserine and CD9 by the test specimen measurement value (value (B)) of the exosome having CD9 as an indicator, the AUC was 0.973, and therefore it was found that Parkinson's disease (particularly Parkinson's disease without cognitive symptoms) can be detected with extremely high accuracy.

Example 6. Evaluation of Parkinson's disease test specimen (MMSE score of 27 or less) using ratio of amount of exosomes having PS and CD9 to amount of exosomes having CD9 as indicator Exosomes were measured by sandwich ELISA of Tim protein-anti-CD9 antibody in the same manner as in Example 1, except that 8 test specimens of patients with Parkinson's disease (MMSE score of 27 or less) and EDTA plasma of 30 test specimens of healthy subjects purchased from PrecisionMed, LLC. were used, and "test specimen measurement value" (value (A)) were obtained.

In addition, exosomes were measured by sandwich ELISA of anti-CD9 antibody-anti-CD9 antibody in the same manner as in Comparative Example 1, except that 8 test specimens of patients with Parkinson's disease (MMSE score of 27 or less) and EDTA plasma of 30 test specimens of healthy subjects purchased from PrecisionMed, LLC. were used, and "test specimen measurement value" (value (B)) were obtained.

The "test specimen measurement value" (value (A)) was divided by the "test specimen measurement value" (value (B)) to obtain (A)/(B) (hereinafter, referred to as "corrected test specimen measurement value"). Based on the obtained corrected test specimen measurement value, a significant difference test between a patient with Parkinson's disease (MMSE score of 27 or less) and a healthy subject was carried out in the same manner as in Example 1 (4), and the AUC and p-value were calculated.

The obtained results are shown in Table 2 which will be given later. In addition, Fig. 3 shows a box plot graph created based on the corrected test specimen measurement value. In the figure, the vertical axis shows the test specimen measurement value, and Control on the horizontal axis shows the results of a healthy subject, PD (MMSE score of 27 or less) on the horizontal axis shows the results of a patient with Parkinson's disease (MMSE score of 27 or less), and PD (MMSE score of greater than 27) on the horizontal axis shows the results of a patient with Parkinson's disease (MMSE score of greater than 27).

**Table 2**

| | Specimen | Solid phase | Detection | Test specimen measurement value used for analysis | Healthy subject-PD (MMSE score of 27 or less 27) | |
|---|---|---|---|---|---|---|
| | | | | | AUC | p-value |
| Example 6 | Plasma | Tim4 | αCD9 | Value (A) | - | - |
| | | αCD9 | αCD9 | Value (B) | - | - |
| | | | | Value (A)/Value (B) | 0.804 | 0.0094 |

### Example 7. Evaluation of Parkinson's disease test specimen (MMSE score of greater than 27) using amount of exosomes having PS and CD9 as indicator

Exosomes were measured by sandwich ELISA of Tim protein-anti-CD9 antibody in the same manner as in Example 1, except that 8 test specimens of patients with Parkinson's disease (MMSE score of greater than 27) and EDTA plasma of 30 test specimens of healthy subjects purchased from PrecisionMed, LLC. were used. A significant difference test between a patient with Parkinson's disease (MMSE score of greater than 27) and a healthy subject was carried out, and the AUC and p-value were calculated.

The obtained results are shown in Table 3 which will be given later.

### Comparative Example 4. Evaluation of Parkinson's disease test specimen (MMSE score of greater than 27) using amount of exosomes having CD9 as indicator

Exosomes were measured by sandwich ELISA of anti-CD9 antibody-anti-CD9 antibody in the same manner as in Comparative Example 1, except that 8 test specimens of patients with Parkinson's disease (MMSE score of greater than 27) and EDTA plasma of 30 test specimens of healthy subjects purchased from PrecisionMed, LLC. were used. A significant difference test between a patient with Parkinson's disease (MMSE score of greater than 27) and a healthy subject was carried out, and the AUC and p-value were calculated.

The obtained results are shown in Table 3 which will be given later.

Example 8. Evaluation of Parkinson's disease test specimen (MMSE score of greater than 27) using ratio of amount of exosomes having PS and CD9 to amount of exosomes having CD9 as indicator

The "test specimen measurement value" (value (A)) obtained in Example 7 (sandwich ELISA of Tim protein-anti-CD9 antibody) was divided by the "test specimen measurement value" (value (B)) obtained in Comparative Example 4 (sandwich ELISA of anti-CD9 antibody-anti-CD9 antibody) to obtain (A)/(B) (hereinafter, referred to as "corrected test specimen measurement value").

Based on the obtained corrected test specimen measurement value, a significant difference test between a patient with Parkinson's disease (MMSE score of greater than 27) and a healthy subject was carried out in the same manner as in Example 1 (4), and the AUC and p-value were calculated.

The obtained results are shown in Table 3 which will be given later. In addition, Fig. 3 shows a box plot graph created based on the corrected test specimen measurement value.

**Table 3**

| | Specimen | Solid phase | Detection | Test specimen measurement value used for analysis | Healthy subject-PD (MMSE score of greater than 27) | |
|---|---|---|---|---|---|---|
| | | | | | AUC | p-value |
| Example 7 | Plasma | Tim4 | αCD9 | Value (A) | - | 0.0001 |
| Comparative Example 4 | | αCD9 | αCD9 | Value (B) | - | 0.0008 |
| Example 8 | | | | Value (A)/Value (B) | 0.950 | 0.0001 |

Example 9. Evaluation of Parkinson's disease test specimens (MMSE score of 27 or less) and Parkinson's disease test specimen (MMSE score of greater than 27) using ratio of amount of exosomes having PS and CD9 to amount of exosomes having CD9 as indicator

Exosomes were measured by sandwich ELISA of Tim protein-anti-CD9 antibody in the same manner as in Example 1, except that 8 test specimens of patients with Parkinson's disease (MMSE score of 27 or less) purchased from PrecisionMed, LLC. and 8 test specimens of patients with Parkinson's disease (MMSE score of greater than 27) purchased from PrecisionMed, LLC. were used, and a "test specimen measurement value" (value (A)) was obtained.

In addition, exosomes were measured by sandwich ELISA of anti-CD9 antibody-anti-CD9 antibody in the same manner as in Comparative Example 1, except that 8 test specimens of patients with Parkinson's disease (MMSE score of 27 or less) purchased from PrecisionMed, LLC. and 8 test specimens of patients with Parkinson's disease (MMSE score of greater than 27) purchased from PrecisionMed, LLC. were used, and a "test specimen measurement value" (value (B)) was obtained.

The "test specimen measurement value" (value (A)) was divided by the "test specimen measurement value" (value (B)) to obtain (A)/(B) (hereinafter, referred to as "corrected test specimen measurement value"). Based on the obtained corrected test specimen measurement value, a significant difference test between a patient with Parkinson's disease (MMSE score of 27 or less) and a Parkinson's disease test specimen (MMSE score of greater than 27) was carried out in the same manner as in Example 1 (4), and the AUC and p-value were calculated.

The obtained results are shown in Table 4 which will be given later. In addition, Fig. 3 shows a box plot graph created based on the corrected test specimen measurement value.

**Table 4**

| | Specimen | Solid phase | Detection | Test specimen measurement value used for analysis | PD (MMSE score of 27 or less)-PD (MMSE score of greater than 27 | |
|---|---|---|---|---|---|---|
| | | | | | AUC | p-value |
| Example 9 | Plasma | Tim4 | αCD9 | Value (A) | - | - |
| | | αCD9 | αCD9 | Value (B) | - | - |
| | | | | Value (A)/Value (B) | 0.844 | 0.0239 |

### Example 10. Evaluation of Parkinson's disease test specimen using amount of exosomes having PS and CD9 as indicator

Exosomes were measured by sandwich ELISA of Tim protein-anti-CD9 antibody in the same manner as in Example 1, except that 16 test specimens of patients with Parkinson's disease (8 test specimens with MMSE score of 27 or less and 8 test specimens with MMSE score of greater than 27) and EDTA plasma of 30 test specimens of healthy subjects purchased from PrecisionMed, LLC. were used. A significant difference test between a patient with Parkinson's disease and a healthy subject was carried out, and the AUC and p-value were calculated.

The obtained results are shown in Table 5 which will be given later. In addition, Fig. 4 shows a box plot graph created based on the test specimen measurement value. In the figure, the vertical axis shows the test specimen measurement value, and Control on the horizontal axis shows the results of a healthy subject, and PD on the horizontal axis shows the results of a patient with Parkinson's disease.

### Comparative Example 7. Evaluation of Parkinson's disease test specimen using amount of exosome having CD9 as indicator

Exosomes were measured by sandwich ELISA of anti-CD9 antibody-anti-CD9 antibody in the same manner as in Comparative Example 1, except that 16 test specimens of patients with Parkinson's disease (8 test specimens with MMSE score of 27 or less and 8 test specimens with MMSE score of greater than 27) and EDTA plasma of 30 test specimens of healthy subjects purchased from PrecisionMed, LLC. were used. A significant difference test between a patient with Parkinson's disease and a healthy subject was carried out, and the AUC and p-value were calculated.

The obtained results are shown in Table 5 which will be given later.

### Example 11. Evaluation of Parkinson's disease test specimen using ratio of amount of exosomes having PS and CD9 to amount of exosomes having CD9 as indicator

The "test specimen measurement value" (value (A)) obtained in Example 10 (sandwich ELISA of Tim protein-anti-CD9 antibody) was divided by the "test specimen measurement value" (value (B)) obtained in Comparative Example 7 (sandwich ELISA of anti-CD9 antibody-anti-CD9 antibody) to obtain (A)/(B) (hereinafter, referred to as "corrected test specimen measurement value").

Based on the obtained corrected test specimen measurement value, a significant difference test between a patient with Parkinson's disease and a healthy subject was carried out in the same manner as in Example 1 (4), and the AUC and p-value were calculated.

The obtained results are shown in Table 5 which will be given later. In addition, Fig. 5 shows a box plot graph created based on the test specimen measurement value. In the figure, the vertical axis shows the corrected test specimen measurement value, and Control on the horizontal axis shows the results of a healthy subject, and PD on the horizontal axis shows the results of a patient with Parkinson's disease.

**Table 5**

| | Specimen | Solid phase | Detection | Test specimen measurement value used for analysis | Healthy subject-PD | |
|---|---|---|---|---|---|---|
| | | | | | AUC | p-value |
| Example 10 | Plasma | Tim4 | αCD9 | Value (A) | 0.769 | 0.003 |
| Comparative Example 7 | | αCD9 | αCD9 | Value (B) | 0.723 | 0.014 |
| Example 11 | | | | Value (A)/Value (B) | 0.877 | <0.0001 |

From Table 2, in a case where the corrected test specimen measurement value ((A)/(B)) obtained by dividing the test specimen measurement value (value (A)) of the exosome having phosphatidylserine and CD9 by the test specimen measurement value (value (B)) of the exosome having CD9 was evaluated using plasma derived from a patient with Parkinson's disease with cognitive symptoms (MMSE score of 27 or less) and a healthy subject as the specimen, the AUC was 0.804, and therefore it was found that Parkinson's disease (particularly Parkinson's disease with cognitive symptoms) can be detected with high accuracy.

From Table 3, in a case where the corrected test specimen measurement value ((A)/(B)) obtained by dividing the test specimen measurement value (value (A)) of the exosome having phosphatidylserine and CD9 by the test specimen measurement value (value (B)) of the exosome having CD9 was evaluated using plasma derived from a patient with Parkinson's disease without cognitive symptoms (MMSE score of greater than 27) and a healthy subject as the specimen, the AUC was 0.950, and therefore it was found that Parkinson's disease (particularly Parkinson's disease without cognitive symptoms) can be detected with extremely high accuracy.

From Table 4, in a case where the corrected test specimen measurement value ((A)/(B)) obtained by dividing the test specimen measurement value (value (A)) of the exosome having phosphatidylserine and CD9 by the test specimen measurement value (value (B)) of the exosome having CD9 was evaluated using plasma of a patient with Parkinson's disease with cognitive symptoms (MMSE score of 27 or less) and a patient with Parkinson's disease without cognitive symptoms (MMSE score of greater than 27) as the specimen, the AUC was 0.844, and therefore it was found that a patient with Parkinson's disease with cognitive symptoms (MMSE score of 27 or less) and a patient with Parkinson's disease without cognitive symptoms (MMSE score of greater than 27) can be distinguished and detected with high accuracy. In addition, the correlation coefficient between the corrected test specimen measurement value ((A)/(B)) and the MMSE was 0.7833, and therefore a high correlation was observed.

From Table 5, in a case where Parkinson's disease was evaluated using plasma derived from a patient with Parkinson's disease (including a patient with Parkinson's disease with cognitive symptoms and a patient with Parkinson's disease without cognitive symptoms) and a healthy subject as the specimen and using the amount of exosomes having CD9 as an indicator, the AUC was 0.764, and in a case where the corrected test specimen measurement value ((A)/(B)) obtained by dividing the test specimen measurement value (value (A)) of the exosome having phosphatidylserine and CD9 by the test specimen measurement value (value (B)) of the exosome having CD9 was evaluated, the AUC was 0.877, and therefore it was found that Parkinson's disease can be detected with high accuracy.

## Claims

1. A method for assisting the diagnosis of Parkinson's disease, comprising:
measuring an amount of extracellular vesicles having phosphatidylserine and tetraspanin, or the amount of extracellular vesicles having phosphatidylserine and tetraspanin and an amount of extracellular vesicles having tetraspanin, in a biological specimen derived from a subject; and
determining that the subject has Parkinson's disease using the amount of extracellular vesicles having phosphatidylserine and tetraspanin, or a ratio of the amount of extracellular vesicles having phosphatidylserine and tetraspanin to the amount of extracellular vesicles having tetraspanin as an indicator,
wherein the determination of Parkinson's disease is carried out in such a manner that the subject is determined to have Parkinson's disease in a case where the amount of extracellular vesicles having phosphatidylserine and tetraspanin, or the ratio of the amount of extracellular vesicles having phosphatidylserine and tetraspanin to the amount of extracellular vesicles having tetraspanin is equal to or less than a reference value.

2. The method for assisting the diagnosis of Parkinson's disease according to claim 1,
wherein the measurement of the amount of extracellular vesicles is to measure the amount of extracellular vesicles having phosphatidylserine and tetraspanin,
the determination of Parkinson's disease is to determine that the subject has Parkinson's disease using the amount of extracellular vesicles having phosphatidylserine and tetraspanin as an indicator, and
the tetraspanin is selected from CD9, CD63, and CD81.

3. The method for assisting the diagnosis of Parkinson's disease according to claim 1,
wherein the measurement of the amount of extracellular vesicles is to measure the amount of extracellular vesicles having phosphatidylserine and tetraspanin,
the measurement of the amount of extracellular vesicles having phosphatidylserine and tetraspanin is to measure the amount of extracellular vesicles having phosphatidylserine and tetraspanin using a substance having an affinity for tetraspanin and a substance having an affinity for phosphatidylserine, and
the determination of Parkinson's disease is to determine that the subject has Parkinson's disease using the amount of extracellular vesicles having phosphatidylserine and tetraspanin as an indicator.

4. The method for assisting the diagnosis of Parkinson's disease according to claim 3,
wherein the substance having an affinity for phosphatidylserine is a T-cell immunoglobulin-mucin-containing protein.

5. The method for assisting the diagnosis of Parkinson's disease according to claim 1,
wherein the measurement of the amount of extracellular vesicles is to measure the amount of extracellular vesicles having phosphatidylserine and tetraspanin,
the determination of Parkinson's disease is to determine that the subject has Parkinson's disease using the amount of extracellular vesicles having phosphatidylserine and tetraspanin as an indicator, and
the biological specimen is a blood specimen or a cerebrospinal fluid.

6. The method for assisting the diagnosis of Parkinson's disease according to claim 1,
wherein the measurement of the amount of extracellular vesicles is to measure the amount of extracellular vesicles having phosphatidylserine and tetraspanin, and the amount of extracellular vesicles having tetraspanin,
the determination of Parkinson's disease is to determine that the subject has Parkinson's disease using the ratio of the amount of extracellular vesicles having phosphatidylserine and tetraspanin to the amount of extracellular vesicles having tetraspanin as an indicator, and
the tetraspanin is CD9 or CD63.

7. The method for assisting the diagnosis of Parkinson's disease according to claim 1,
wherein the measurement of the amount of extracellular vesicles is to measure the amount of extracellular vesicles having phosphatidylserine and tetraspanin, and the amount of extracellular vesicles having tetraspanin,
the measurement of the amount of extracellular vesicles having phosphatidylserine and tetraspanin is to measure the amount of extracellular vesicles having phosphatidylserine and tetraspanin using a substance having an affinity for tetraspanin and a substance having an affinity for phosphatidylserine,
the measurement of the amount of extracellular vesicles having tetraspanin is to measure the amount of extracellular vesicles having tetraspanin using the substance having an affinity for tetraspanin, and
the determination of Parkinson's disease is to determine that the subject has Parkinson's disease using the ratio of the amount of extracellular vesicles having phosphatidylserine and tetraspanin to the amount of extracellular vesicles having tetraspanin as an indicator.

8. The method for assisting the diagnosis of Parkinson's disease according to claim 7,
wherein the substance having an affinity for phosphatidylserine is a T-cell immunoglobulin-mucin-containing protein.

9. The method for assisting the diagnosis of Parkinson's disease according to claim 1,
wherein the measurement of the amount of extracellular vesicles is to measure the amount of extracellular vesicles having phosphatidylserine and tetraspanin, and the amount of extracellular vesicles having tetraspanin,
the determination of Parkinson's disease is to determine that the subject has Parkinson's disease using the ratio of the amount of extracellular vesicles having phosphatidylserine and tetraspanin to the amount of extracellular vesicles having tetraspanin as an indicator, and
the biological specimen is a blood specimen or a cerebrospinal fluid.

10. The method for assisting the diagnosis of Parkinson's disease according to claim 1,
wherein the determination of Parkinson's disease is to determine whether the subject has Parkinson's disease with cognitive symptoms or Parkinson's disease without cognitive symptoms using the ratio of the amount of extracellular vesicles having phosphatidylserine and tetraspanin to the amount of extracellular vesicles having tetraspanin as an indicator.

11. Use of a reagent kit for the method for assisting the diagnosis of Parkinson's disease according to any one of claims 1 to 10, comprising:
a substance having specific affinity for tetraspanin; and
a substance having specific affinity for phosphatidylserine.

12. Use of an extracellular vesicle having phosphatidylserine and tetraspanin as biomarker in the method for assisting the diagnosis of Parkinson's disease according to any one of claims 1 to 10.

## Patentansprüche

1. Verfahren zur Unterstützung der Diagnose der Parkinson-Krankheit, umfassend:
Messen einer Menge extrazellulärer Vesikel mit Phosphatidylserin und Tetraspanin, oder der Menge extrazellulärer Vesikel mit Phosphatidylserin und Tetraspanin und einer Menge extrazellulärer Vesikel mit Tetraspanin, in einer von einem Subjekt stammenden biologischen Probe; und
Bestimmen, dass das Subjekt die Parkinson-Krankheit aufweist, unter Verwendung der Menge extrazellulärer Vesikel mit Phosphatidylserin und Tetraspanin oder eines Verhältnisses der Menge extrazellulärer Vesikel mit Phosphatidylserin und Tetraspanin zu der Menge extrazellulärer Vesikel mit Tetraspanin als Indikator,
wobei die Bestimmung der Parkinson-Krankheit derart durchgeführt wird, dass bestimmt wird, dass das Subjekt die Parkinson-Krankheit aufweist, wenn die Menge extrazellulärer Vesikel mit Phosphatidylserin und Tetraspanin oder das Verhältnis der Menge extrazellulärer Vesikel mit Phosphatidylserin und Tetraspanin zu der Menge extrazellulärer Vesikel mit Tetraspanin gleich einem Referenzwert oder kleiner als dieser ist.

2. Verfahren zur Unterstützung der Diagnose der Parkinson-Krankheit nach Anspruch 1,
wobei die Messung der Menge extrazellulärer Vesikel das Messen der Menge extrazellulärer Vesikel mit Phosphatidylserin und Tetraspanin ist,
die Bestimmung der Parkinson-Krankheit das Bestimmen ist, dass das Subjekt die Parkinson-Krankheit aufweist, unter Verwendung der Menge extrazellulärer Vesikel mit Phosphatidylserin und Tetraspanin als Indikator, und
das Tetraspanin aus CD9, CD63 und CD81 ausgewählt ist.

3. Verfahren zur Unterstützung der Diagnose der Parkinson-Krankheit nach Anspruch 1,
wobei die Messung der Menge extrazellulärer Vesikel das Messen der Menge extrazellulärer Vesikel mit Phosphatidylserin und Tetraspanin ist,
die Messung der Menge extrazellulärer Vesikel mit Phosphatidylserin und Tetraspanin das Messen der Menge extrazellulärer Vesikel mit Phosphatidylserin und Tetraspanin unter Verwendung einer Substanz mit Affinität zu Tetraspanin und einer Substanz mit Affinität zu Phosphatidylserin ist, und
die Bestimmung der Parkinson-Krankheit das Bestimmen ist, dass das Subjekt die Parkinson-Krankheit aufweist, unter Verwendung der Menge extrazellulärer Vesikel mit Phosphatidylserin und Tetraspanin als Indikator.

4. Verfahren zur Unterstützung der Diagnose der Parkinson-Krankheit nach Anspruch 3, wobei die Substanz mit Affinität zu Phosphatidylserin ein T-Zell-Immunglobulin-Mucinenthaltendes Protein ist.

5. Verfahren zur Unterstützung der Diagnose der Parkinson-Krankheit nach Anspruch 1,
wobei die Messung der Menge extrazellulärer Vesikel das Messen der Menge extrazellulärer Vesikel mit Phosphatidylserin und Tetraspanin ist,
die Bestimmung der Parkinson-Krankheit das Bestimmen ist, dass das Subjekt die Parkinson-Krankheit aufweist, unter Verwendung der Menge extrazellulärer Vesikel mit Phosphatidylserin und Tetraspanin als Indikator, und
die biologische Probe eine Blutprobe oder eine Zerebrospinalflüssigkeit ist.

6. Verfahren zur Unterstützung der Diagnose der Parkinson-Krankheit nach Anspruch 1,
wobei die Messung der Menge extrazellulärer Vesikel das Messen der Menge extrazellulärer Vesikel mit Phosphatidylserin und Tetraspanin und der Menge extrazellulärer Vesikel mit Tetraspanin ist,
die Bestimmung der Parkinson-Krankheit das Bestimmen ist, dass das Subjekt die Parkinson-Krankheit aufweist, unter Verwendung des Verhältnisses der Menge extrazellulärer Vesikel mit Phosphatidylserin und Tetraspanin zu der Menge extrazellulärer Vesikel mit Tetraspanin als Indikator, und
das Tetraspanin CD9 oder CD63 ist.

7. Verfahren zur Unterstützung der Diagnose der Parkinson-Krankheit nach Anspruch 1,
wobei die Messung der Menge extrazellulärer Vesikel das Messen der Menge extrazellulärer Vesikel mit Phosphatidylserin und Tetraspanin und der Menge extrazellulärer Vesikel mit Tetraspanin ist,
die Messung der Menge extrazellulärer Vesikel mit Phosphatidylserin und Tetraspanin das Messen der Menge extrazellulärer Vesikel mit Phosphatidylserin und Tetraspanin unter Verwendung einer Substanz mit Affinität zu Tetraspanin und einer Substanz mit Affinität zu Phosphatidylserin ist,
die Messung der Menge extrazellulärer Vesikel mit Tetraspanin das Messen der Menge extrazellulärer Vesikel mit Tetraspanin unter Verwendung der Substanz mit Affinität zu Tetraspanin ist, und
die Bestimmung der Parkinson-Krankheit das Bestimmen ist, dass das Subjekt die Parkinson-Krankheit aufweist, unter Verwendung des Verhältnisses der Menge extrazellulärer Vesikel mit Phosphatidylserin und Tetraspanin zu der Menge extrazellulärer Vesikel mit Tetraspanin als Indikator.

8. Verfahren zur Unterstützung der Diagnose der Parkinson-Krankheit nach Anspruch 7,
wobei die Substanz mit Affinität zu Phosphatidylserin ein T-Zell-Immunglobulin-Mucin-enthaltendes Protein ist.

9. Verfahren zur Unterstützung der Diagnose der Parkinson-Krankheit nach Anspruch 1,
wobei die Messung der Menge extrazellulärer Vesikel das Messen der Menge extrazellulärer Vesikel mit Phosphatidylserin und Tetraspanin und der Menge extrazellulärer Vesikel mit Tetraspanin ist,
die Bestimmung der Parkinson-Krankheit das Bestimmen ist, dass das Subjekt die Parkinson-Krankheit aufweist, unter Verwendung des Verhältnisses der Menge extrazellulärer Vesikel mit Phosphatidylserin und Tetraspanin zu der Menge extrazellulärer Vesikel mit Tetraspanin als Indikator, und die biologische Probe eine Blutprobe oder eine Zerebrospinalflüssigkeit ist.

10. Verfahren zur Unterstützung der Diagnose der Parkinson-Krankheit nach Anspruch 1,
wobei die Bestimmung der Parkinson-Krankheit das Bestimmen ist, ob das Subjekt die Parkinson-Krankheit mit kognitiven Symptomen oder die Parkinson-Krankheit ohne kognitive Symptome aufweist, unter Verwendung des Verhältnisses der Menge extrazellulärer Vesikel mit Phosphatidylserin und Tetraspanin zu der Menge extrazellulärer Vesikel mit Tetraspanin als Indikator.

11. Verwendung eines Reagenzienkits für das Verfahren zur Unterstützung der Diagnose der Parkinson-Krankheit nach einem der Ansprüche 1 bis 10, umfassend:
eine Substanz mit Affinität zu Tetraspanin; und
eine Substanz mit Affinität zu Phosphatidylserin.

12. Verwendung eines Biomarkers für das Verfahren zur Unterstützung der Diagnose der Parkinson-Krankheit nach einem der Ansprüche 1 bis 10, umfassend:
ein extrazelluläres Vesikel mit Phosphatidylserin und Tetraspanin.

## Revendications

1. Procédé d'assistance au diagnostic de la maladie de Parkinson, comprenant les étapes suivantes :
mesurer une quantité de vésicules extracellulaires présentant de la phosphatidylsérine et de la tétraspanine, ou la quantité de vésicules extracellulaires présentant de la phosphatidylsérine et de la tétraspanine et une quantité de vésicules extracellulaires présentant de la tétraspanine, dans un spécimen biologique dérivé d'un sujet, et
déterminer que le sujet est affecté de la maladie de Parkinson à l'aide de la quantité de vésicules extracellulaires présentant de la phosphatidylsérine et de la tétraspanine, ou un rapport entre la quantité de vésicules extracellulaires présentant de la phosphatidylsérine et de la tétraspanine et la quantité de vésicules extracellulaires présentant de la tétraspanine comme indicateur,
dans lequel la détermination de la maladie de Parkinson est réalisée de telle sorte que le sujet est déterminé comme étant affecté de la maladie Parkinson dans un cas où la quantité de vésicules extracellulaires présentant de la phosphatidylsérine et de la tétraspanine, ou le rapport entre la quantité de vésicules extracellulaires présentant de la phosphatidylsérine et de la tétraspanine et la quantité de vésicules extracellulaires présentant de la tétraspanine est inférieur(e) ou égal(e) à une valeur de référence.

2. Procédé d'assistance au diagnostic de la maladie de Parkinson selon la revendication 1,
dans lequel la mesure de la quantité de vésicules extracellulaires consiste en la mesure de la quantité de vésicules extracellulaires présentant de la phosphatidylsérine et de la tétraspanine, et
la détermination de la maladie de Parkinson consiste en la détermination que le sujet est affecté de la maladie de Parkinson à l'aide de la quantité de vésicules extracellulaires présentant de la phosphatidylsérine et de la tétraspanine comme indicateur, et
la tétraspanine est sélectionnée parmi CD9, CD63, et CD81.

3. Procédé d'assistance au diagnostic de la maladie de Parkinson selon la revendication 1,
dans lequel la mesure de la quantité de vésicules extracellulaires consiste en la mesure de la quantité de vésicules extracellulaires présentant de la phosphatidylsérine et de la tétraspanine ;
la mesure de la quantité de vésicules extracellulaires présentant de la phosphatidylsérine et de la tétraspanine consiste en la mesure de la quantité de vésicules extracellulaires présentant de la phosphatidylsérine et de la tétraspanine à l'aide d'une substance présentant une affinité pour la tétraspanine et une substance présentant une affinité pour la phosphatidylsérine, et
la détermination de la maladie de Parkinson consiste en la détermination que le sujet est affecté de la maladie de Parkinson à l'aide de la quantité de vésicules extracellulaires présentant de la phosphatidylsérine et de la tétraspanine comme indicateur.

4. Procédé d'assistance au diagnostic de la maladie de Parkinson selon la revendication 3,
dans lequel la substance présentant une affinité pour la phosphatidylsérine est une protéine contenant de l'immunoglobuline-mucine de lymphocyte T.

5. Procédé d'assistance au diagnostic de la maladie de Parkinson selon la revendication 1,
dans lequel la mesure de la quantité de vésicules extracellulaires consiste en la mesure de la quantité de vésicules extracellulaires présentant de la phosphatidylsérine et de la tétraspanine ;
la détermination de la maladie de Parkinson consiste en la détermination que le sujet est affecté de la maladie de Parkinson à l'aide de la quantité de vésicules extracellulaires présentant de la phosphatidylsérine et de la tétraspanine comme indicateur, et
le spécimen biologique est un échantillon de sang ou un liquide cérébrospinal.

6. Procédé d'assistance au diagnostic de la maladie de Parkinson selon la revendication 1,
dans lequel la mesure de la quantité de vésicules extracellulaires consiste en la mesure de la quantité de vésicules extracellulaires présentant de la phosphatidylsérine et de la tétraspanine et de la quantité de vésicules extracellulaires présentant de la tétraspanine ;
la détermination de la maladie de Parkinson consiste en la détermination que le sujet est affecté de la maladie de Parkinson à l'aide du rapport entre la quantité de vésicules extracellulaires présentant de la phosphatidylsérine et de la tétraspanine et la quantité de vésicules extracellulaires présentant de la tétraspanine comme indicateur, et
la tétraspanine est CD9 ou CD63.

7. Procédé d'assistance au diagnostic de la maladie de Parkinson selon la revendication 1,
dans lequel la mesure de la quantité de vésicules extracellulaires consiste en la mesure de la quantité de vésicules extracellulaires présentant de la phosphatidylsérine et de la tétraspanine et de la quantité de vésicules extracellulaires présentant de la tétraspanine ;
la mesure de la quantité de vésicules extracellulaires présentant de la phosphatidylsérine et de la tétraspanine consiste en la mesure de la quantité de vésicules extracellulaires présentant de la phosphatidylsérine et de la tétraspanine à l'aide d'une substance présentant une affinité pour la tétraspanine et une substance présentant une affinité pour la phosphatidylsérine ;
la mesure de la quantité de vésicules extracellulaires présentant de la tétraspanine consiste en la mesure de la quantité de vésicules extracellulaires présentant de la tétraspanine à l'aide d'une substance présentant une affinité pour la tétraspanine, et
la détermination de la maladie de Parkinson consiste en la détermination que le sujet est affecté de la maladie de Parkinson à l'aide du rapport entre la quantité de vésicules extracellulaires présentant de la phosphatidylsérine et de la tétraspanine et la quantité de vésicules extracellulaires présentant de la tétraspanine comme indicateur.

8. Procédé d'assistance au diagnostic de la maladie de Parkinson selon la revendication 7,
dans lequel la substance présentant une affinité pour la phosphatidylsérine est une protéine contenant de l'immunoglobuline-mucine de lymphocyte T.

9. Procédé d'assistance au diagnostic de la maladie de Parkinson selon la revendication 1,
dans lequel la mesure de la quantité de vésicules extracellulaires consiste en la mesure de la quantité de vésicules extracellulaires présentant de la phosphatidylsérine et de la tétraspanine et de la quantité de vésicules extracellulaires présentant de la tétraspanine ;
la détermination de la maladie de Parkinson consiste en la détermination que le sujet est affecté de la maladie de Parkinson à l'aide du rapport entre la quantité de vésicules extracellulaires présentant de la phosphatidylsérine et de la tétraspanine et la quantité de vésicules extracellulaires présentant de la tétraspanine comme indicateur, et
le spécimen biologique est un échantillon de sang ou un liquide cérébrospinal.

10. Procédé d'assistance au diagnostic de la maladie de Parkinson selon la revendication 1,
dans lequel la détermination de la maladie de Parkinson consiste à déterminer que le sujet est affecté de la maladie de Parkinson avec troubles cognitifs ou de la maladie de Parkinson sans troubles cognitifs à l'aide du rapport entre la quantité de vésicules extracellulaires présentant de la phosphatidylsérine et de la tétraspanine et la quantité de vésicules extracellulaires présentant de la tétraspanine comme indicateur.

11. Utilisation d'un kit de réactif pour le procédé d'assistance au diagnostic de la maladie de Parkinson selon l'une quelconque des revendications 1 à 10, comprenant :
une substance présentant une affinité spécifique pour la tétraspanine, et
une substance présentant une affinité spécifique pour la phosphatidylsérine.

12. Utilisation d'une vésicule extracellulaire présentant de la phosphatidylsérine et de la tétraspanine comme biomarqueur dans le procédé d'assistance au diagnostic de la maladie de Parkinson selon l'une quelconque des revendications 1 à 10.
